# EUROPEAN PATENT SPECIFICATION

(11) **EP 1 531 790 B1**
(45) Date of publication and mention of the grant of the patent: **20.10.2010**
(21) Application number: 03736683.8
(22) Date of filing: 12.06.2003
(51) Int. Cl.: A61K 8/89, A61K 31/74, A61Q 19/00

(54) **COSMETIC COMPOSITION STRUCTURED WITH SILICONE POLYMERS AND FILM-FORMING RESINS**
MIT SILIKONPOLYMEREN UND FILMBILDENDEN HARZEN STRUKTURIERTE KOSMETISCHE ZUSAMMENSETZUNG
COMPOSITION COSMETIQUE STRUCTUREE AVEC DES POLYMERES DE SILICONE ET DES RESINES FILMOGENES

(30) Priority: 12.06.2002 US 166755
(43) Date of publication of application: 25.05.2005
(73) Proprietor: L'Oréal, 75008 Paris (FR)
(72) Inventor: YU, Wei, Edison, NJ 08820 (US)
(74) Representative: Le Coupanec, Pascale A.M.P.
(86) International application number: PCT/US2003/016209
(87) International publication number: WO 2003/105801

(56) References cited:
- EP-A- 1 266 648
- EP-A- 1 266 653
- WO-A-02/17870
- WO-A-99/06473
- US-A- 5 919 441
- US-A- 6 060 072

## Description

### Technical field

This invention relates to a cosmetic composition for care and/or treatment and/or makeup of the skin, including the scalp and/or the lips of humans, containing a liquid fatty phase comprising at least one silicone oil gelled with a special polymer, existing notably in the form of a cast makeup product, in particular of a makeup stick such as lipsticks, the application of which results in a glossy and non-migrating deposit.

A cosmetic composition for care and/or treatment is a composition which comprises at least one active compound for treating wrinkles; for moisturizing the skin and the lips, for protecting the skin, the lips and the phaneric structures from ultraviolet rays, for treating acne and/or for acting as a self-tanning preparation.

The invention relates most especially to cosmetic and dermatological compositions such as makeup products, possessing properties of staying power, but also of non-transfer and stability.

### State of the prior art

In cosmetic or dermatological products, it is common to find a structured, that is, gelled and/or rigidified, liquid fatty phase; this is especially the case in cast solid compositions such as deodorants, lip balms and lipsticks, eye shadows, concealers, and foundations. This structuring is obtained with the aid of waxes or fillers. Unfortunately these waxes and fillers have a tendency to make the composition matte, which is not always desirable, in particular for a lipstick or an eye shadow.

In the meaning of the application, by liquid fatty phase is understood a fatty phase which is liquid at room temperature (25° C) and atmospheric pressure (760 mmHg), consisting of one or more fatty substances which are liquid at room temperature, also referred to as oils, mutually compatible and containing one silicone oil.

In the meaning of the application, by structured liquid fatty phase it is understood that this structured phase does not flow under its own weight.

The structuring of the liquid fatty phase makes it possible in particular to limit its exudation from solid compositions and, in addition, after deposition on the skin or the lips, to limit the migration of this phase into wrinkles and fine lines, which is particularly sought for a lipstick or an eye shadow. In fact, a significant migration of the liquid fatty phase, charged with coloring agents, leads to an unsightly appearance around the lips or the eyes, in particular accentuating wrinkles and fine lines. This migration often is cited by women as a major drawback of conventional lipsticks or eye shadows. By migration is understood an extension of the composition deposited on the skin or the lips beyond its initial border.

Gloss basically is associated with the nature of the liquid fatty phase. Thus it is possible to reduce the level of waxes and fillers in the composition in order to increase the gloss of a lipstick, but then the migration of the liquid fatty phase increases. In other words, the levels of waxes and of fillers required for the making of a stick of suitable hardness place a restriction on the gloss of the deposit.

Document EP-A-1 068 856 [1] describes solid cosmetic compositions, without wax, comprising a liquid fatty phase structured with a polymer, in which the fatty phase is mainly a non-silicone oil.

Document WO-A-01/97758 [2] describes cosmetic compositions based on polyamide resins comprising a gelling agent chosen from among the esters and amides of N-acylamino acids and mixtures thereof. The composition also comprises a polyamide resin solvent which may be chosen from among the saturated or unsaturated fatty alcohols, the esters of fatty and/or aromatic carboxylic acids, the ethoxylated and/or propoxylated alcohols and acids, the silicones, the mineral oils and the branched-chain hydrocarbons; preferably, the esters of fatty acids, the fatty alcohols, the mineral oils, the branched hydrocarbons and mixtures of the latter.

The use of fatty phases based on silicone oils has made it possible up to now to obtain cosmetic compositions having a long staying power when the oils are not very volatile or are nonvolatile, that is, a good staying power particularly of color in the course of time (non-changing, non-fading), and non-transfer compositions when the silicone oils are volatile, not becoming deposited on a support such as a glass, a cup, a fabric or a cigarette, placed in contact with the film of makeup.

At the present time, the use of silicone oils in cosmetics is limited by the lack of molecules capable of gelling these mediums and thus resulting in compositions existing in solid form such as lipsticks or cast foundations, for example. The utilization of cosmetic compositions the fatty phase of which is mainly silicone in most cases results in problems of compatibility with the ingredients traditionally used in the cosmetics industry.

In documents US-A-5,874,069 [3], US-A-5,919,441 [4], US-A-6,051,216 [5], WO-A-02/17870 [6], and WO-A-02/17871 [7], WO-A-99/06473 [12], US-A-6,353,076 [13], cosmetic compositions such as deodorant sticks or gels were achieved comprising a silicone oily phase gelled with a wax based on polysiloxane and polyamide, or with a polymer containing siloxane groups and groups capable of hydrogen interactions.

In WO-A-02/17870 [6], adding another gelling agent to the composition is contemplated, but the quantities added are to be small, for example less than 0.5% in the case of hydroxystearic acid, to preserve the clearness of the product.

In WO-A-02/17871 [7], using a second gelling agent with the silicone polymer in a quantity representing 0.5 to 2% by weight of the composition, and a solvent system comprising a non-silicone organic compound, a volatile silicone and possibly another silicone also is contemplated.

Document EP-A-1 177 784 [8] illustrates a deodorant composition comprising a liquid phase containing, for example, a volatile silicone and possibly a nonvolatile silicone and/or a non-silicone hydrophobic organic liquid, structured with an organic compound with amido groups, possibly with one or more polymer or non-polymer secondary structuring agents, in smaller proportions. Among the secondary structuring agents, this document mentions the polymers having siloxane groups and groups with hydrogen interactions without giving examples or results on a composition using these polymers.

Documents EP-A-1 266 653 [14] and EP-A-1 266 648 [15] relate to physiologically acceptable cosmetic compositions comprising a liquid fatty phase comprising at least one silicone oil and being structured with at least one polymer with a weight-average molecular mass ranging from 500 to 500 000, containing at least one moiety comprising: at least one polyorganosiloxane group, composed of 1 to 1000 organosiloxane units in the chain of the moiety or in the form of a graft, and at least two groups capable of establishing hydrogen Interactions, the polymer being solid at room temperature and soluble in the liquid fatty phase at a temperature of 25°C to 250°C. EP-A-1 266 653 also comprises solid particles having a hydrophobic membrane, when EP-A-1 266 646 [15] comprises solid particles and at least one amphiphilic silicone.

In document US-A-6 060 072 [16], the invention relates to transfer-resistant color cosmetic compositions comprising a film forming agent, a volatile oil, a styrene-ethylene-propylene copolymer as gellant, and optionally, a pigment.

It should be noted that documents [6], [7] and [8] relate to deodorants for which problems of exudation and migration of the liquid fatty phase charged with a coloring agent into the wrinkles and fine lines, as well as staying power and non-transfer of the composition do not arise as in the case of the makeup cosmetic products described hereinabove. In addition, gloss is not sought for deodorants.

Moreover, the sticks obtained by structuring the liquid fatty phase solely with one or more gelling silicone polymers do not afford a sufficient mechanical resistance to shearing, particularly when the stick is applied on the lips and/or the skin, resulting in a breaking of the stick.

### Statement of the invention

The purpose of the invention is precisely to provide a composition for care and/or makeup and/or treatment of the skin and/or the lips, making it possible to remedy the drawbacks and to solve the problems mentioned previously; in particular the purpose of the invention is to provide a composition having improved deposit staying-power properties.

In a surprising manner, the applicant has found that the use of special polymers combined with one or more film-forming silicone resins made it possible, in the absence of wax, to structure the silicone-oil-based liquid fatty phases in the form of a makeup or care product the application of which resulted in a non-migrating glossy, satiny or matte film in accordance with the wish of the user and/or the type of product sought, and to intensify the staying-power and, possibly, non-transfer properties of these products. In addition, their thermal stability and their slip in application are improved.

By stable is understood a composition which does not exude at room temperature (25° C) for at least 2 months, or even up to 9 months.

The combination of these special polymers with one or more film-forming silicone resins makes it possible to obtain gels, in particular solid gels, having a good mechanical resistance and a correct rheology for allowing a deposit in sufficient quantity which is not sticky to the touch, affords a very good staying power, does not transfer (in particular when volatile silicone oils are used) and does not migrate into wrinkles and fine lines.

The effects obtained by virtue of this combination of a special polymer and a special silicone resin, notably the surprising improvement in the staying power of the deposit obtained, do not appear in the document of the prior art.

The invention applies not only to products for making up the lips, such as lipsticks, lip pencils, lip glosses, but also to products for care and/or treatment of the skin, including the scalp, and the lips, such as the products in stick form for solar protection of the skin, the face or the lips, or lip balms, to products for making up the skin, of the face as well as of the human body, such as foundations cast in a stick or a dish, concealer products and products for temporary tattooing, to products for cleansing, especially in stick form, and to products for making up the eyes, such as eyeliners, in particular in pencil form, and mascaras, especially cakes for the keratinous fibers (eyelashes, eyebrows, hair).

More precisely, the purpose of the invention is a cosmetic composition for care and/or makeup, comprising:
1) a liquid fatty phase comprising at least one silicone oil, structured with at least one gelling system comprising at least one polymer (homopolymer or copolymer) with a weight-average molecular mass ranging from 500 to 500,000, containing at least one moiety comprising:
   - at least one polyorganosiloxane group, composed of 1 to 1000 organosiloxane units in the chain of the moiety or in the form of a graft, and
   - at least two groups capable of establishing hydrogen interactions, said groups being amide groups of formula -C(O)NH- and -HC-C(O)-
   - the polymer being solid at room temperature and soluble in the liquid fatty phase at a temperature of 25 to 250° C, and
2) at least one film-forming silicone resin, the liquid fatty phase, the gelling system, and the film-forming silicone resin forming a physiologically acceptable medium, said polymer comprising at least one moiety of formula: (III) : 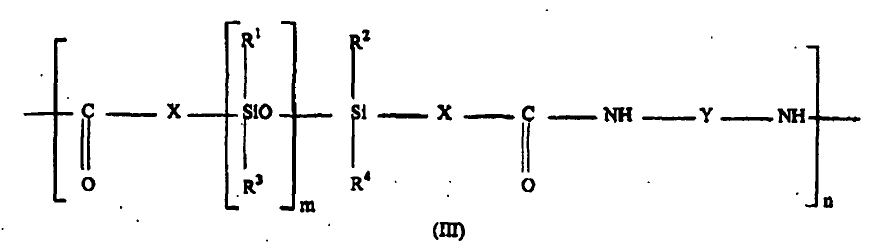 where :
   - m is in the range from 1 to 700, preferably from 15 to. 500 and n in particular is in the range from 1 to 500, preferable from 1 to 100 and better still from 4 to 25,
   - X is a linear or branched alkylene chain having 1 to 30 carbon atoms, in particular 3 to 10 carbon atoms,
   - Y is an alkylene chain that is linear or branched or that can comprise rings and/or unsaturations, having from 1 to 40 carbon atoms, in particular from 1 to 20 carbon atoms, and better still from 2 to 6 carbon atoms, in particular 6 carbon atoms, and
   - R¹, R², R³ and R⁴ represent, independently, a linear or branched, C1 to C40 alkyl group, preferably a CH3, C2H5, n-C3H7 or isopropyl group, a polyorganosiloxane chain or a phenyl group possibly substituted with one to three methyl or ethyl groups,
      said cosmetic composition being further characterized in that the film-forming silicone resin is a trimethylsiloxysilicate comprising repeating moieties of formula

      [(CH₃)₃-Si-O]ₓ-(SiO_{4/2})=Y (MQ)

      in which x ranges from 50 to 80 and y ranges from 50 to 80,
      and said cosmetic composition further comprising a colouring agent.

According to the invention, by "gelling system" generally is understood a system making it possible to rigidify the composition by formation of hydrogen bonds.

The composition of the invention can exist in the form of a paste, a solid, a more or less viscous cream. It can be a simple or multiple emulsion, in particular oil-in-water or water-in-oil, water-in-oil-in-water or oil-in-water-in-oil, or a rigid or supple gel with an oily continuous phase. The simple or multiple emulsion can comprise an aqueous or oily continuous phase possibly containing dispersed lipid vesicles. In particular it exists in a form cast in a stick or a dish and more especially in the form of a rigid oily gel in particular anhydrous and notably an anhydrous stick. More especially, it exists in the form of a rigid translucent or opaque gel (depending on whether or not it contains pigments), the liquid fatty phase forming the continuous phase, An anhydrous composition will comprise less than 10% by weight of water, for example less than 5% by weight.

The structuring of the liquid fatty phase can be modified depending on the nature of the polymer and of the film-forming silicone resin used in the gelling system, and can be such that a rigid structure in the form of a wand or stick, with good mechanical resistance, is obtained. These wands, when they are colored, make it possible, after application, to obtain a glossy, non-migrating deposit with good staying power, especially of the color, over time. The composition can contain one or more structuring polymers and one or more film-forming silicone resins.

The composition of the invention advantageously is a composition for the lips and better a lipstick composition especially in stick form.

### Liquid fatty phase

According to the invention, the liquid fatty phase comprises at least one silicone oil which can be a volatile oil, a nonvolatile oil or a mixture of volatile oil(s) and nonvolatile oil(s).

An oil is a non-aqueous compound immiscible in water.

The liquid fatty phase preferably comprises at least one volatile silicone oil.

According to the invention, the volatile silicone oil can be chosen from among the linear or cyclic silicone oils having a flash point equal to or in excess of 40° C and advantageously in excess of the softening point of the polymer of the gelling system and/or a viscosity less than 8 cSt, such as the linear or cyclic polydimethylsiloxanes (PDMS) having from 3 to 7 silicon atoms.

The flash point is the temperature at which a fuel ignites on contact with a flame.

As examples of such oils, there may be cited the compounds mentioned in Table 1 below.

The volatile oil advantageously has a flash point in excess of 60° C.

The nonvolatile silicone oils can be polydimethylsiloxanes, polyalkylmethylsiloxanes, dimethicone copolyols, alkylmethicone copolyols, cetyldimethicone, silicones with alkylglyceryl ether groups, silicones with side amine groups and dilauroyltrimethylol propane siloxysilicate. The alkyl groups of these oils have in particular from 2 to 24 carbon atoms.

The non-volatile silicone oils which can be used in the invention can be, in particular, non-volatile linear polydimethylsiloxanes (PDMS) liquid at room temperature; polydimethylsiloxanes containing alkyl, alcoxy or phenyl groups, pendent and/or at the end of the silicone chain, groups each having from 2 to 24 carbon atoms; phenylated silicones such as the phenyl trimethicones, the phenyl dimethicones, the phenyl trimethylsiloxy diphenylsiloxanes, the diphenyl dimethicones, the diphenyl methyldiphenyl trisiloxanes, the 2-phenylethyl trimethylsiloxysilicates, the fluorous silicones with pendent or end-chain group(s) having from 1 to 12 carbon atoms in which all or part of the hydrogen atoms are substituted with fluorine atoms, the dimethiconols and mixtures thereof.

According to the invention, the liquid fatty phase can comprise at least one volatile silicone oil and at least one volatile non-silicone oil.

In the meaning of the invention, a volatile silicone or non-silicone oil preferably has a flash point of 40 to 135° C or no flash point. At room temperature (25° C) and atmospheric pressure (760 mmHg), the volatile oils have a vapor pressure ranging from 0.02 mm to 300 mmHg (2.66 Pa to 40,000 Pa), and better ranging from 0.1 to 90 mmHg (13 Pa to 12,000 Pa). The non-volatile oils then correspond to a vapor pressure less than 0.02 mmHg (2.66 Pa).

The silicone oils of the invention have a viscosity advantageously chosen in the range from 5 to 800,000 cSt at 25° C, preferably from 10 to 500,000 cSt, and better from 10 to 5,000 cSt.

**Table 1**

| **Compound** | **Flash point (°C)** | **viscosity (cSt)** |
|---|---|---|
| Octyltrimethicone | 93 | 1.2 |
| | | |
| Hexyltrimethicone | 79 | 1.2 |
| | | |
| Decamethyl cyclopentasiloxane (cyclopentasiloxane or D5) | 72 | 4.2 |
| | | |
| Octamethylcyclo tetrasiloxane (cyclotetradimethyl siloxane or D4) | 55 | 2.5 |
| | | |
| Dodecamethylcyclo hexasiloxane (D6) | 93 | 7 |
| | | |
| Decamethyltetrasiloxane (L4) | 63 | 1.7 |
| | | |
| KF 96 A from Shin Etsu | 94 | 6 |
| | | |
| PDMS (polydimethylsiloxane) DC 200 (1.5 cSt) from Dow Corning | 56 | 1.5 |
| | | |
| PDMS DC 200 (2 cSt) from Dow Corning | 87 | 2 |
| | | |
| PDMS DC 200 (5 cSt) from Dow Coming | 134 | 5 cSt |
| | | |
| PDMS DC 200 (3 cSt) from Dow Coming | 102 | 3 cSt |

In other words, the volatile silicone oil(s) may be chosen, for example, from within the group composed of the compounds of Table 1, heptamethyloctyltrisiloxane, dodecamethylpentasiloxane and mixtures thereof.

The volatile silicone oil also can be chosen from within the group of fluorous silicone oils such as the silicones with alkyl and perfluoralkyl groups, the silicones with oxyethylene/oxypropylene (OE/PP) side groups and perfluorous groups, the silicones with perfluorous side groups and glycerol side groups, and the perfluoroalkylmethylphenylsiloxanes, these oils having a vapor pressure in excess of or equal to 0.02 mmHg.

According to the invention, the liquid fatty phase can contain one or more volatile or nonvolatile non-silicone oils. The volatile non-silicone oils can be chosen from within the group of hydrocarbon oils and volatile esters and ethers such as the volatile hydrocarbons like isododecane and isohexadecane, the C₈-C₁₆ isoparaffins, the isohexyl or isodecyl neopentanoates.

The volatile non-silicone oil also may be chosen from among the fluorous oils such as the perfluoropolyethers, the perfluoroalkanes such as perfluorodecaline, the perfluorodamantanes, the monoesters, diesters and triesters of perfluoroalkyl-phosphates and the fluorous ester oils.

As examples of volatile non-silicone oils which can be used in the invention, there may be cited the compounds of Table 2 which follows.

**Table 2**

| **Compound** | **Flash point (°C)** |
|---|---|
| Isododecane | 43 |
| Isohexadecane | 102 |
| Isodecyl neopentanoate | 118 |
| propylene glycol n-butylether | 60 |
| 3-ethyl ethoxypropionate | 58 |
| Propylene glycol methylether acetate* | 46 |
| Isopar L (C₁₁-C₁₃ isoparaffin) | 62 |
| Isopar H (C₁₁-C₁₂ isoparaffin) | 56 |

The liquid fatty phase advantageously contains at least 30% and better still at least 40% by weight of silicone oil(s) advantageously having a viscosity of less than 1,000 cSt and better less than 100 cSt, because the silicone polymers used in the invention are more soluble in the low-viscosity silicone oils. It also can contain other oils or a mixture of non-silicone oils.

When the fatty phase comprises a volatile oil, the latter advantageously represents from 3 to 89.4%, and better from 5 to 60%, for example from 5 to 10% of the total weight of the composition.

The liquid fatty phase also can contain other non-silicone oils, for example polar oils such as:
- the hydrocarbon vegetable oils with a high triglyceride content composed of esters of fatty acids and glycerol, the fatty acids of which can have varying chain lengths, the latter being able to be linear or branched, saturated or unsaturated; these oils are in particular wheat germ oil, corn oil, sunflower oil, karite butter, castor oil, sweet almond oil, macadamia oil, apricot oil, soybean oil, rape oil, cotton oil, alfalfa oil, poppy oil, pumpkin oil, sesame oil, marrow oil, avocado oil, hazelnut oil, grapeseed or blackcurrant seed oil, evening primrose oil, millet oil, barley oil, quinoa oil, olive oil, rye oil, safflower oil, candlenut oil, passion flower oil musk rose oil; or else caprylic/capric acid triglycerides such as those sold by the company Stearines Dubois or those sold under the names Miglyol 810, 812 and 818 by the company Dynamit Nobel;
- the synthetic oils or esters of formula R₅COOR₆ in which R₅ represents the residue of a linear or branched higher fatty acid containing from 1 to 40 and better from 7 to 19 carbon atoms and R₆ represents a branched hydrocarbon chain containing from 1 to 40 and better from 3 to 20 carbon atoms, with R₅ + R₆ ≥ 10, such as, for example, purcellin oil (cetostearyl octanoate), isononyl isononanoate, C₁₂ to C₁₅ alkyl benzoate, isopropyl myristate, 2-ethylhexyl palmitate, alkyl or polyalkyl octanoates, decanoates or ricineolates; hydroxylated esters such as isostearyl lactate, diisostearyl malate; and pentaerythritol esters;
- synthetic ethers having from 10 to 40 carbon atoms;
- C₈ to C₂₆ fatty alcohols such as oleic alcohol or octyldodecanol;
- fatty acids such as oleic acid, linoleic acid or linolenic acid; and
- mixtures thereof.

The liquid fatty phase also can contain apolar oils such as linear or branched hydrocarbons or fluorocarbons of synthetic or mineral origin, volatile or nonvolatile, such as volatile paraffin oils (such as isoparaffins, isododecane) or nonvolatile paraffin oils and derivatives thereof, vaseline, polydecanes, hydrogenated polyisobutene such as parleam, squalane, and mixtures thereof.

Thus the invention can be implemented, for example, with the following different fatty phases:
1) a fatty phase consisting of a mixture of oils comprising at least one nonvolatile silicone oil and at least one volatile silicone oil;
2) a fatty phase consisting of a mixture of oils comprising at least one nonvolatile silicone oil and at least one volatile non-silicone oil;
3) a fatty phase consisting of a mixture of oils comprising at least one nonvolatile silicone oil, at least one volatile silicone oil and at least one volatile non-silicone oil;
4) a fatty phase consisting of a mixture of oils comprising at least one volatile silicone oil, one nonvolatile non-silicone oil and possibly at least one volatile non-silicone oil, and
5) a fatty phase consisting solely of volatile silicone oil(s).

In cases 1), 2) and 3), the mixture also can comprise a nonvolatile non-silicone oil.

The liquid fatty phase generally represents from 5 to 99% of the total weight of the composition, and better from 20 to 75%.

### Solid particles

According to the invention, the composition generally furthermore comprises solid particles chosen from among fillers and pigments (including nacreous pigments), and mixtures thereof.

Generally the average size of the solid particles is from 10 nm to 50 µm, and better from 50 nm to 30 µm, for example from 100 nm to 10 µm.

The fillers used in cosmetic compositions generally have the purpose of absorbing sweat and sebum and/or imparting, a matte appearance. According to the invention, they furthermore make it possible to structure the liquid fatty phase comprising a silicone oil and to intensify the properties of staying power and/or non-transfer of the composition as well as the thermal stability.

By pigments is understood any solid particle insoluble in the composition serving to impart and/or to modify a color and/or an iridescent appearance.

These pigments can ensure both the function of absorption of sweat and sebum, and the function of coloring or of modification of the appearance of the composition, that is, of the makeup and/or care cosmetic product. In the invention, they also ensure the structuring of the liquid fatty phase.

These fillers or pigments can be of either hydrophobic or hydrophilic nature. When these fillers or pigments are hydrophilic particles, their dispersion in the composition is facilitated either by coating them in a film of hydrophobic compound or by adding a dispersant, and in particular a silicone amphiphilic with respect to the composition.

The hydrophobic pigments or fillers, can be composed of hydrophobic polymer or copolymer powders. As examples of hydrophobic polymers and copolymers used as fillers, there may be cited:
1) fluorous polymers such as polytetrafluoroethylene powders and powders of tetrafluoroethylene and olefin copolymer, for example of ethylene or propylene; 2) silicone elastomers, for example polymethylsilsesquioxane powders (Tospearl^{®} from Toshiba); 3) polyolefins such as polyethylene; 4) alkyl polymethacrylates, for example methyl polymethacrylate; 5) polyamides (Nylon^{®}); 6) polystyrenes; 7) polyesters and derivatives thereof; 8) polyacrylics (Polytrap^{®} from Dow Corning) or methyl polymethacrylate; and 9) polyurethanes, for example powders of Hexamethylene Diisocyanate/trimethylol hexalactone.

There also can be used hydrophilic fillers treated on the surface so as to be hydrophobic, such as boron nitride, starch, precipitated calcium carbonate, silica, glass or a ceramic.

Instead of powders, there of course can be used fibers of a hydrophobic nature, in particular fibers of the polymers and copolymers cited previously.

The solid particles also can be composed of pigments and/or nacres making it possible to obtain a covering makeup, that is, not allowing the skin, lips or phaneric structures to show through. These particles furthermore make it possible to reduce the sticky feel of the compositions.

The pigments can be white or colored, organic and/or inorganic, coated or uncoated. The inorganic pigments can be chosen, for example, from among the zinc oxides, iron oxides, titanium oxides and mixtures thereof. Thus among the inorganic pigments, there may be cited titanium or zinc dioxide, possibly surface-treated, zirconium or cerium oxides, as well as iron or chromium oxides, manganese violet, ultramarine blue, chromium hydrate and ferric blue. Among the organic pigments there may be cited carbon black, pigments of the D & C type, and the lakes based on cochineal carmine, on barium, strontium, calcium, aluminum. The pigments can represent from 0.1 to 50%, preferably from 0.5 to 40% and better from 2 to 30% of the total weight of the composition, if they are present.

The nacreous pigments (or nacres) can be chosen from among the white nacres such as mica coated with titanium or with bismuth oxychloride, colored nacreous pigments such as titanium mica with iron oxides, titanium mica with, in particular, ferric blue or chromium oxide, titanium mica with an organic pigment of the type mentioned above, as well as nacreous pigments based on bismuth oxychloride. The nacreous pigments also can have gogniochromatic properties and exist in the form of liquid crystals or multilayered plaques. They can represent from 0 to 30%, preferably from 0.1 to 20% of the total weight of the composition, and better from 0.1 to 15%.

When the pigments or the fillers are hydrophilic, they are coated in a film of hydrophobic compound with a view to introducing them into the liquid fatty phase of the composition of the invention.

The coating can be a fluorous coating such as a perfluoroalkyl mono- or diester of phosphoric acid (acid or salt), a perfluoropolyether, a carboxylic or sulfonic perfluoroacid, or a salt of diethanolamine perfluoroalkyl phosphate.

The coating can be a coating based on fluorous silicone, for example a coating-grafting with a silane with a perfluoroalkyl group.

The coating also can be implemented by means of silicone derivatives, for example a coating-grafting with reactive silicones initially possessing hydrogenosilane groups, a coating grafting with a diorganosilane such as dimethylchlorosilane or with an alkylalcoxysilane, a coating-grafting with a silane with a glycydoxypropyl group, a coating with a polyglycerol silicone , or a coating with a silicone or silicone-g-polyacrylic grafted acrylic copolymer.

There also can be used a coating with N-acylamino acids, for example N-lauroyllysine, coatings with fatty acids or fatty acid salts of the stearic acid type, coatings with lecithins and coatings with ester oils.

Dispersion of the hydrophilic particles also may be facilitated by means of at least one amphiphilic silicone which serves as a surfactant between the hydrophilic particles and the hydrophobic silicone phase.

These amphiphilic silicones contain a silicone portion which is compatible with the highly silicone medium of the compositions of the invention, and a hydrophilic portion which can be, for example, the residue of a compound chosen from among the alcohols and the polyols, having from 1 to 12 hydroxyl groups, the polyoxyalkylenes containing at least two oxyalkylene moieties and having from 0 to 20 oxypropylene moieties and/or from 0 to 20 oxyethylene moieties. This hydrophilic portion therefore has an affinity for the hydrophilic particles and contributes to the dispersion thereof in the silicone medium.

The amphilic silicone can be an oil without gelling activity. Such oils can be composed of:
- dimethicone copolyols, possibly containing phenyl groups,
- alkylmethicone copolyols,
- polyglycerol silicones, that is, silicones with alkylglyceryl ether groups,
- silicones with perfluorous side groups and with glycerol side groups,
- silicones with polyoxyethylene/polyoxypropylene side groups and with perfluorous side groups,
- silicone block and hydrophilic block copolymers other than polyether, for example polyoxazoline or polyethyleneimine,
- grafted copolymers of the silicone grafted polysaccharide type,
- silicone block, poly(ethylene oxide/propylene oxide) block copolymers.

The amphiphilic silicone used in the invention also can be an at least partially crosslinked amphiphilic silicone resin.

As examples of such resins, there can be cited:
- crosslinked silicon resins with alkylpolyether groups, such as ethylene polyoxide (POE) and ethylene polyoxide/propylene polyoxide (POE/POP), described in US-A-5,412,004 [9], and
- silicone resins crosslinked in part with α,ω-dienes, possessing both hydrophilic POE/POP side chains and hydrophobic alkyl side chains such as those described in EP-A-1 048 686 [10]. The hydrophilic side chains are obtained by reaction with a POE/POP at a single vinyl end, and the alkyl side chains are formed by reaction with an α-olefin with a fatty chain.

In the amphiphilic silicone resin, the silicone portion advantageously is made up of polydimethylsiloxane.

### Gelling silicone polymer

The polymer or polymers structuring or gelling the composition is/are solid at room temperature (25° C) and atmospheric pressure (760 mmHg) and soluble in the liquid fatty phase at a temperature of 25 to 250° C.

In the meaning of the invention, there is understood by polymer a compound having at least 2 repeating moieties, preferably at least 3 repeating moieties, and better still 10 repeating moieties.

In the composition of the invention, the silicone polymer of the gelling system represents generally from 0.5 to 80%, preferably from 2 to 60%, and better still from 5 to 40% of the total weight of the composition.

Furthermore, the gelling-system polymer/silicone oil(s) mass ratio is preferably from 0.1 to 50%.

The polymers used as gelling agents in the composition of the invention are polymers of the polyorganosiloxane type such as those described in the documents US-A-5,874,069 [3], US-A-5,919,441 [4], US-A-6,051,216 [5] and US-A-5,981,680 [11].

According to the invention, the polymers used as a gelling agent can belong to the following two families:
1) polyorganosiloxanes comprising at least two groups capable of establishing hydrogen interactions, these two groups being located in the polymer chain; and/or
2) polyorganosiloxanes comprising at least two groups capable of establishing hydrogen interactions, these two groups being located on grafts or branches.

The polymers to which the invention applies are solids that can be dissolved beforehand in a solvent with hydrogen interaction capable of breaking the hydrogen interactions of the polymers, such as the C₂ to C₈ lower alcohols and in particular ethanol, n-propanol, isopropanol, before being placed in the presence of the silicone oils according to the invention. It also is possible to use these hydrogen interaction "breaking" solvents as cosolvent. These solvents then can be kept in the composition or else be removed by selection evaporation, well known to the person skilled in the art.

According to the invention, the polymer used as a gelling agent can be a homopolymer, that is, a polymer comprising several identical moieties.

According to the invention, the groups capable of establishing hydrogen interactions are amide groups of formula -C(O)NH- and -HN-C(O)-.

In this first embodiment of the invention, the gelling agent also can consist of a grafted copolymer. Thus the polyamide with silicone units can be grafted and possibly crosslinked with silicone chains having amide groups. Such polymers can be synthesized with trifunctional amines.

In this case, the copolymer can comprise at least one moiety of formula: 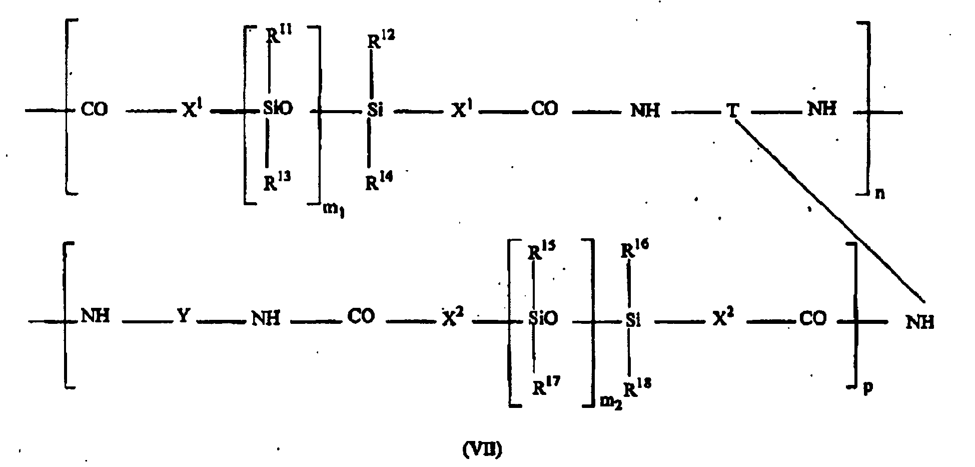 in which X¹ and X², which are identical or different,
represent a linear or branched C₁ to C₃₀ alkylenediyl group, which can contain in its chain one or more oxygen and/or nitrogen atoms
n is a integer ranging from 2 to 500, preferably from 2 to 200
Y is a saturated or unsaturated, C₁ to C₃₀ linear or branched divalent alkylene, arylene, cycloalkylene, alkylarylene or arylalkylene group, which can comprise one or more oxygen, sulfur and/or nitrogen atoms, and/or bear as substituent one of the following atoms or groups of atoms: fluorine, hydroxy, C₃ to C₆ cycloalkyl, C₁ to C₄₀ alkyl, C₅ to C₁₀ aryl, phenyl possibly substituted with 1 to 3 C₁ to C₃ alkyl groups, C₁ to C₃ hydroxyalkyl and C₁ to C₆ aminoalkyl, or
4) Y represents a group corresponding to the formula: 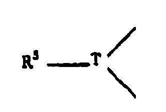 in which
- T represents a linear or branched, saturated or unsaturated, C₃ to C₂₄ trivalent or tetravalent hydrocarbon chain, possible substituted with a polyorganosiloxane chain, and which can contain one or more atoms chosen from among O, N and S, or T represents a trivalent atom chosen from among N, P and Al, and
- R⁵ repreaents a linear or branched C₁ to C₃₀ alkyl group, or a polyorganosiloxane chain, which can comprise one or more eater, amide, urethane, thiocarbamate, urea, thiourea and/or sulfonamide groups which can be linked or not linked to another chain of the polymer,
R¹¹ to R¹⁸ are groups chosen from within the same group as R¹ to R⁴, R¹, R², R³ and R⁴, identical or different, represent a group chosen from among:
- linear, branched or cyclic, saturated or unsaturated, C₁ to C₄₀ hydrocarbon groups, which can contain in their chain one or more oxygen, sulfur and/or nitrogen atoms, and which can be partly or totally substituted with fluorine atoms,
- C₅ to C₁₀ aryl groups, possibly substituted with one or more C₁ to C₄ alkyl groups,
- polyorganosiloxane chains containing or not containing one or more oxygen, sulfur and/or nitrogen atoms;
m₁ and m₂ are numbers falling in the range from 1 to 1000, and p is an integer ranging from 2 to 500.

In formula (VII), it is preferred that:
- p is in the range of 1 to 25, better still from 1 to 7,
- R¹¹ to R¹⁸ are methyl groups,
- T corresponds to one of the following formulas: 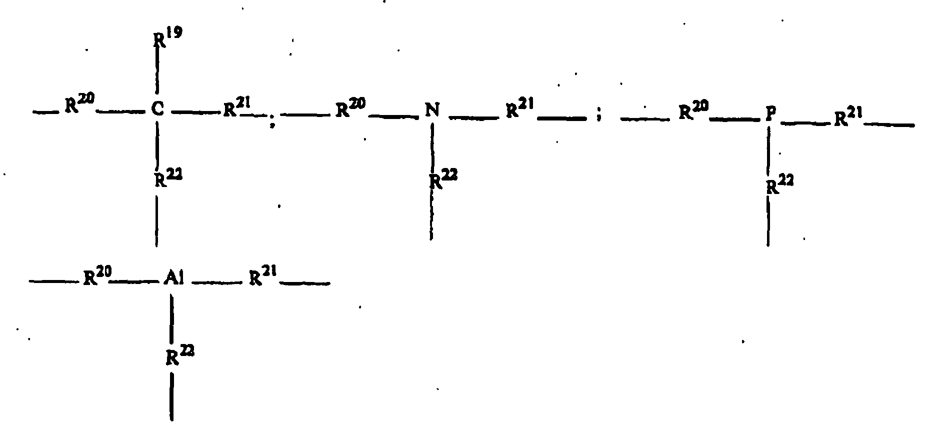 in which R¹⁹ is a hydrogen atom or a group chosen from among the groups defined for R¹ to R⁴, and R²⁰, R²¹ and R²² are, independently, linear or branched alkylene groups, and preferably still, with the formula: 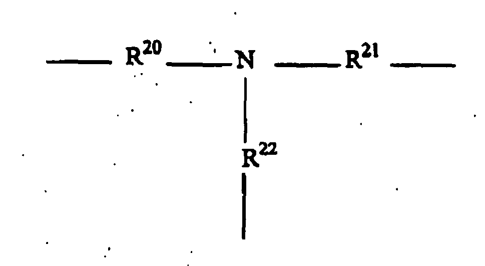 in particular with R²⁰, R²¹ and R²² representing -CH₂-CH₂-,
- m₁ and m₂ are in the range from 15 to 500, and better still from 15 to 45,
- X¹ and X² represent -(CH₂)₁₀-, and
- Y represents -CH₂-.

These polyamides with a grafted silicone moiety of formula (VII) can be copolymerized with polyamide-silicones of formula (II) to form block copolymers, alternating copolymers or random copolymers. Formula (II) : 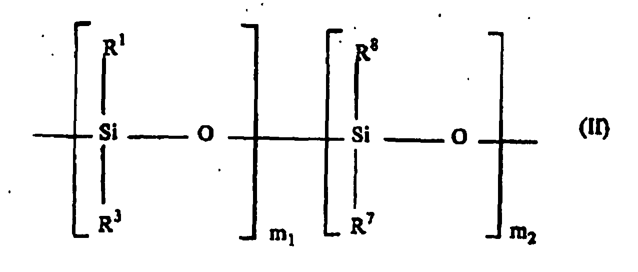 in which
- R¹ and R³, identical or different, are such as defined hereinabove for formula (I),
- R⁷ represents a group such as defined hereinabove for R¹ and R³, or represents the group of formula -X-G-R⁹ in which X and G are such as defined hereinabove for formula (I) and R⁹ represents a hydrogen atom or a linear, branched or cyclic, saturated or unsaturated, C₁ to C₅₀ hydrocarbon group, possibly comprising in its chain one or more atoms chosen from among O, S and N, possibly substituted with one or more fluorine atoms and/or one or more hydroxyl groups, or a phenyl group possibly substituted with one or more C₁ to C₄ alkyl groups.
- R⁸ represents the group of formula -X-G-R⁹ in which X, G and R⁹ are such as defined hereinabove,
- m₁ is an integer ranging from 1 to 998, and
- m₂ is an integer ranging from 2 to 500.
The weight percentage of grafted silicone moieties (VII) in the copolymer can range from 0.5% to 30% by weight.

According to the invention, the siloxane units can be in the main chain or backbone of the polymer, but they also can be present in grafted or pendent chains. In the main chain, the siloxane units can be in the form of segments as described hereinabove. In the pendent or grafted chains, the siloxane units can appear individually or in segments.

According to the invention, the preferred siloxane-based polyamides are:
- polyamide of formula (III) in which m is from 15 to 50;
- mixtures of two or more polyamides in which at least one polyamide has a value of m in the range from 15 to 50 and at least one polyamide has a value of m in the range from 30 to 50;
- mixtures of polyamide of formula (III) combining
   1) 80 to 99% by weight of a polyamide in which n is equal to 2 to 10, in particular 3 to 6, and
   2) 1 to 20% of a polyamide in which n is in the range from 5 to 500, in particular from 6 to 100;
      - polyamides of formula (III) synthesized with at least one portion of an activated diacid (diacid chloride, dianhydride or diester) instead of the diacid;
      - polyamides of formula (III) in which X represents -(CH₂)₃- or -(CH₂)₁₀; and
      - polyamides of formula (III) in which the polyamides are terminated with a monofunctional chain chosen from within the group consisting of monofunctional amines, monofunctional acids, monofunctional alcohols, including fatty acids, fatty alcohols, and fatty amines, such as, for example, octylamine, octanol, stearic acid and stearyl alcohol.

According to the invention, the ends of the polymer chains can be terminated with:
- a C₁ to C₅₀ alkyl ester group by introducing a C₁ to C₅₀ monoalcohol during synthesis,
- a C₁ to C₅₀ alkylamide group by taking as stopping group a monoacid if the silicone is α,ω-diaminated, or a monoamine if the silicone is an α,ω-dicarboxylic acid.

According to one embodiment variant of the invention, there can be used a copolymer of silicone polyamide and of hydrocarbon polyamide, that is, a copolymer comprising moieties of formula (III) and hydrocarbon polyamide moieties. In this case, the polyamide-silicone moieties can be arranged at the ends of the hydrocarbon polyamide.

Polyamide-based gelling agents containing silicones can be produced by silylic amidation of polyamides based on fatty acid dimer. This approach involves the reaction of free acid sites existing on a polyamide as end sites, with oligosiloxane-morioamines and/or oligosiloxane-diamines (amidation reaction), or alternatively with oligosiloxane alcohols or oligosiloxane diols (esterification reaction). The esterification reaction requires the presence of acid catalysts, as is known in the art. It is desirable for the polyamide having free acid sites, used for the amidation or esterification reaction, to have a relatively high number of acid end groups (for example, polyamides having high acid values, for example from 15 to 20).

For the amidation of the free acid sites of the hydrocarbon polyamides, siloxane diamines with 1 to 300, more particularly 2 to 50, and better still 2, 6, 9.5, 12, 13.5, 23 or 31 siloxane groups, can be used for the reaction with hydrocarbon diamides based on fatty acid dimers. Siloxane diamines having 13.5 siloxane groups are preferred, and the best results are obtained with the siloxane-diamine having 13.5 siloxane groups and polyamides containing high values for carboxylic acid end groups.

The reactions may be carried out in xylene to extract the water produced from the solution by azeotropic distillation, or at higher temperatures (about 180 to 200° C) without solvent. Typically, the efficacy of the amidation and the reaction rates decrease when the siloxane diamine is longer, that is, when the number of siloxane groups is higher. Free amine sites can be blocked after the initial amidation reaction of the diaminosiloxanes by reacting them either with an acid siloxane or with an organic acid such as benzoic acid.

For the esterification of the free acid sites on the polyamides, this can be performed in boiling xylene with about 1% by weight, relative to the total weight of the reagents, of para-toluenesulfonic acid as catalyst.

These reactions carried out on the carboxylic acid end groups of the polyamide lead to the incorporation of silicone moieties only at the ends of the polymer chain.

A polyamide-silicone copolymer also can be prepared using a polyamide with free amine groups, by amidation reaction with a siloxane containing an acid group.

There also can be prepared a gelling agent based on a copolymer between a hydrocarbon polyamide and a silicone polyamide, by transamidation of a polyamide having, for example, an ethylene-diamine constituent, with an oligosiloxane-α,ω-diamine, at high temperature (for example 200 to 300° C), in order to carry out a transamidation such that the ethylenediamine component of the original polyamide is replaced with the oligosiloxane diamine.

The copolymer of hydrocarbon polyamide and of polyamide-silicone also can be a grafted copolymer comprising a hydrocarbon polyamide backbone with pendent oligosiloxane groups.

This can be obtained, for example:
- by hydrosilylation of unsaturated bonds in polyamides based on fatty acid dimers;
- by silylation of the amide groups of a polyamide; or
- by silylation of unsaturated polyamides by means of an oxidation, that is, by oxidizing the unsaturated groups into alcohols or diols, in order to form hydroxyl groups which are reacted with siloxane carboxylic acids or siloxane alcohols. The olefinic sites of the unsaturated polyamides also can be epoxidized and the epoxy groups then can be reacted with siloxane amines or siloxane alcohols.

The gelling agents consisting of homopolymers or copolymers of the invention can have siloxane moieties in the main chain of the polymer and groups capable of establishing hydrogen interactions, either in the main chain of the polymer or at the ends thereof, or on side chains or branches of the main chain. This can correspond to the following five arrangements: 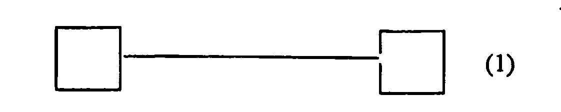 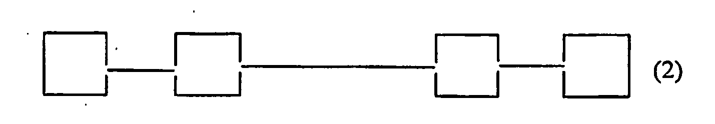 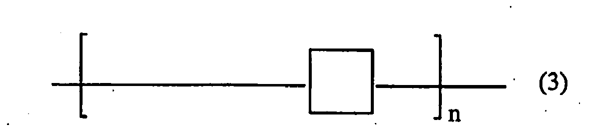 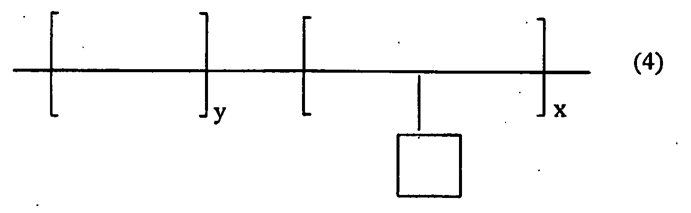 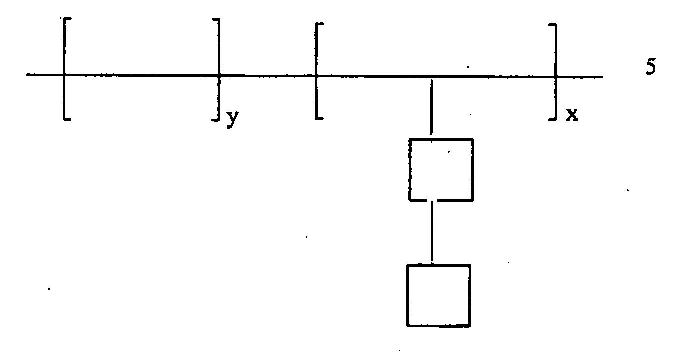 in which the continuous line is the main chain of the siloxane polymer and the squares represent the groups capable of establishing hydrogen interactions.

In case (1), the groups capable of establishing hydrogen interactions are arranged at the ends of the main chain. In case (2), two groups capable of establishing hydrogen interactions are arranged at each of the ends of the main chain.

In case (3), the groups capable of establishing hydrogen interactions are arranged within the main chain in repeating moieties.

In cases (4) and (5), these are copolymers in which the groups capable of establishing hydrogen interactions are arranged on branches of the main chain of a first series of moieties which are copolymerized with moieties not comprising groups capable of establishing hydrogen interactions. The values n, x and y are such that the polymer has the desired properties as a gelling agent for fatty phases based on silicone oil.

According to the invention, the structuring of the liquid fatty phase containing at least one silicone oil is obtained with the aid of one or more of the polymers mentioned above in combination with one or more film-forming silicone resins.

As examples of polymers which can be used, there may be cited the silicone polymers obtained in accordance with Examples 1 and 2 of document US-A-5,981,680.

The polymers and copolymers used in the gelling system for the composition of the invention advantageously have a softening point of 65° C to 190° C. They preferably have a softening point ranging from 70 to 130° C and better from 80° C to 105° C. This softening point is lower than that of the known structuring polymers, which facilitates the use of the polymers which are the subject of the invention, makes possible the use of volatile oils and limits the degradation of the liquid fatty phase.

They have good solubility in silicone oils and result in macroscopically homogeneous compositions. They preferably have an average molecular mass from 500 to 200,000, for example from 1,000 to 100,000, preferably from 2,000 to 30,000.

### Film-forming silicone resin

The composition according to the invention contains one or more film-forming silicone resins. This or these film-forming silicone resin(s) makes/make it possible to improve the staying power and to obtain all the surprising effects mentioned above.

As a general rule, the word "resin" denotes a three-dimensional structure.

The at least one film-forming silicone resin used in the compositions of the invention can be any silicone resin which has film-forming properties.

In one embodiment, the film-forming silicone resin is chosen from among the silsesquioxanes, the siloxysilicates and the resins obtained by hydroxysililation.

The use of silicone polymers or derivatives thereof as film-forming agents in cosmetic compositions is known in the art: see for example, the patents US-A-5,965,112, US-A-5,800,816, US-A-5,911,974 and US-A-5,959,009. Nonetheless, the combination of such resins with the special polymers according to the invention is neither described nor suggested in the prior art. Nothing led one to imagine, in consideration of the prior art, that such a combination could result in a surprising improvement in the properties, especially of staying power and possibly of non-transfer, in a deposit obtained from a cosmetic composition comprising this combination.

The nomenclature of the silicone resins is known in the art under the name of "MDTQ" nomenclature, by which a silicone resin is described according to the various repeating siloxane monomer moieties which constitute the polymer.

Each letter of "MDTQ" corresponds to a different type of moiety.

The symbol "M" corresponds to the monofunctional moiety (CH₃)₃SiO_{1/2}. This moiety is regarded as monofunctional because the silicon atom shares only one oxygen for the formation of the chain. The "M" moiety can be represented by:

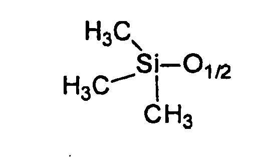

At least one of the methyl groups can be replaced so as, for example, to produce a moiety with the formula [R(CH₃)₂]SiO_{1/2}, such as represented by the following structure: 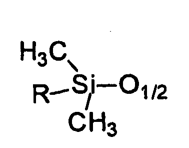 in which R is other than a methyl group.

The symbol "D" corresponds to the difunctional moiety (CH₃)₂SiO_{2/2} in which two of the available bonds on the silicon atom are used to bond with oxygen for the formation of the polymer chain.

The "D" moiety, which is the essential component element of the dimethicone oils, can be represented by:

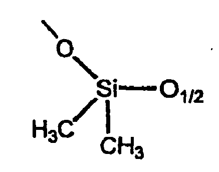

The symbol "T" corresponds to the trifunctional moiety (CH₃)SiO_{3/2}, in which three of the available bonds on the silicon atom are used to bond with oxygen for the formation of the polymer chain.

The "T" moiety can be represented by:

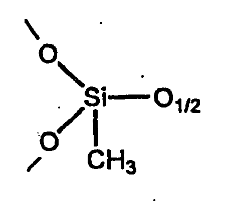

As in the "M" moiety, any one of the methyl groups can be replaced in "D" or "T" by an R group which is other than methyl.

Finally, the symbol "Q" corresponds to a quadrifunctional moiety SiO_{4/2}, in which all four available bonds on the silicon atom are used to bond with oxygen for the formation of the polymer chain.

The "Q" moiety can be represented by:

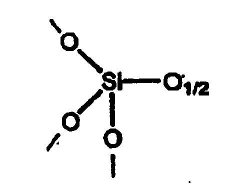

The number of different silicones which can be produced is staggering. It is clear to the person skilled in this field of the art that the properties of'each of the silicones are going to vary according to the type of monomer, the type of substitution, the size of the polymer chain, and the degree of crosslinking or the size of the side chain. Different properties are obtained depending on whether the backbone (the main chain)' is a silicone chain with carbon-based side chains or the backbone is carbon-based with silicone side chains.

The film-forming silicone resins preferably are crosslinked. In one embodiment, the film-forming silicone resin is chosen from among the substituted siloxysilicates, A substituted siloxysilicate can be, for example, a siloxysilicate in which a methyl group has been replaced by'a longer carbon chain, such as an ethane, propane or butane chain. The carbon chain can be saturated or nonsaturated.

The film-forming silicone resin is chosen from among the siloxysilicates, such as the trimethylsiloxysilicates, which are represented by the following formula:

[(CH₃)₃-Si-O]ₓ-(SiO_{4/2})_{y} (MQ moieties),

in which x and y can have values ranging from 50 to 80.

In one embodiment of the invention, these film-forming silicone resins are soluble or dispersible in silicones, in particular volatile, or other organic liquids. In one embodiment, the film-forming silicone resin or resins can be solid at approximately 25° C. They preferably exist in crosslinked or three-dimensional form. In one embodiment, the film-forming silicone resin can have a molecular mass ranging from 1000 to 10,000 grams/mole. In one embodiment, the film-forming silicone resin or resins is/are present in the composition in a quantity ranging from 0.5% to 20% by weight with respect to the total weight of the composition, preferably in a quantity from 1% to 10%. The film-forming silicone resins advantageously comprise one or more hydrophilic groups, imparting emulsifying properties thereto. Under these conditions, the composition can contain an aqueous phase and the resin can function as a dispersant of the aqueous phase in the liquid fatty phase and conversely depending on the ratio between the hydrophilic portion and the hydrophobic (that is, silicone) portion. The resin also can be present only in the aqueous phase.

In order for the resin to be crosslinked, it is necessary that at least one moiety be chosen from T or Q (moiety for crosslinking via a silicon bridge), the other moiety being able to be chosen from M (end moiety) and/or D (non-crosslinked chain fragment moiety).

In another embodiment, the film-forming silicone resin or resins contains/contain repeating M and Q moieties. The ratio of the M moieties to the Q moieties can be, for example, approximately 0.7:1. The at least one film-forming silicone resin can be chosen, for example, from among the WACKER 803 and 804 resins, available from WACKER SILICONE CORPORATION, and G. E. 1,170-002, available from GENERAL ELECTRIC.

The film-forming silicone resin or resins can be chosen from among, for example, the diisostearoyl trimethylolpropane siloxysilicates, such as SF 1,318, available from GE SILICONES.

In addition, irrespective of the type of film-forming silicone resin, the latter can be crosslinked.

Furthermore, irrespective of the type of film-forming silicone resin, crosslinked or non-crosslinked, it possibly also can comprise a hydrophilic group or groups or grafts, in particular of the polyoxyalkylene type, such as the POEs, the PPOs and the POE/PPOs. This means that the film-forming silicone resin also can be represented in the aqueous phase as already has been mentioned above.

The quantities of film-forming silicone resin or resins and of structuring polymer or polymers can be chosen according to the hardness desired and the stability desired in the compositions and according to the specific application contemplated. The respective quantities of the at least one structuring polymer and of the film-forming silicone resin or resins can be such that a solid which can disintegrate and does not flow under the effect of its own weight is obtained.

According to the invention, the polymer can be combined with at least one amphiphilic compound liquid at room temperature, with a hydrophilic/lipophilic balance (HLB) value less than 12, in particular ranging from 1 to 7, preferably from 1 to 5, and better from 3 to 5. According to the invention, one or more amphiphilic compounds may be used. These amphiphilic compounds have the purpose of intensifying the structuring properties of the polymer, facilitating the use of the polymer and improving the depositing capacity of the stick.

According to the invention, the composition preferably has a hardness ranging from 20 to 2,000 gf and better from 20 to 900 gf, in particular from 20 to 600 gf and for example from 150 to 450 gf. This hardness can be measured according to a method of penetration of a probe into said composition and in particular with the aid of a texture analyzer (for example TA-TXT2i from Rheo) equipped with an ebonite cylinder 25 mm in height and 8 mm in diameter. The hardness measurement is performed at 20° C at the center of five samples of said composition. The cylinder is introduced into each sample of composition at a pre-speed of 2 mm/s, then at a speed of 0.5 mm/s and finally at a post-speed of 2mm/s, the total displacement being 1 mm. The recorded hardness value is that of the maximum peak. The measurement error is ± 50 gf.

Hardness also can be measured by the so-called cheesewire method, which consists in cutting a lipstick 12.7 mm in diameter and measuring the hardness at 20° C by means of a DFGHS 2 dynamometer from the company Indelco-Chatillon moving at a speed of 100 mm/minute. It is expressed as the shear force (expressed in gramforce) required to cut a stick under these conditions. According to this method, the hardness of a stick composition according to the invention ranges from 30 to 300 gf, preferably from 30 to 250 gf and for example from 30 to 200 gf, better from 30 to 120 gf, when the diameter of the stick is equal to 12.7 mm.

The hardness of the composition according to the invention is such that the composition is self-supporting and can disintegrate easily to form a satisfactory deposit on the skin and the lips. In addition, with this hardness, the composition of the invention has good impact resistance.

According to the invention, the solid composition, in the form of a stick has the behavior of a deformable, supple elastic solid, imparting a noteworthy elastic softness on application. The stick compositions of the prior art do not have this property of elasticity and suppleness.

The amphiphilic, silicone and non-silicone compound or compounds which can be used in the composition of the invention comprise a lipophilic portion associated with a polar portion, the lipophilic portion comprising a carbon chain having at least 8 carbon atoms, in particular from 18 to 32 carbon atoms, and better from 18 to 28 carbon atoms. The polar portion of this or these amphiphilic compound(s) preferably is the residue of a compound chosen from among the alcohols and polyols having 1 to 12 hydroxyl groups, the polyoxyalkylenes comprising at least two oxyalkylene moieties and having from 0 to 20 oxypropylene moieties and/or from 0 to 20 oxyethylene moieties. In particular, the amphiphilic compound is an ester chosen from among the hydroxystearates; the oleates; the glycerol, sorbitan or methylglucose isostearates; or even C₁₂ to C₂₆ branched fatty alcohols such as octyldodecanol and mixtures thereof. Among these esters, monoesters and mixtures of mono- and diesters are preferred.

The respective levels of film-forming silicone resin and structuring silicone polymer and possibly of amphiphilic compound are chosen according to the desired hardness of the gel and in terms of the specific application contemplated. The respective quantities of polymer, of film-forming silicone resin and possibly of amphiphilic compound are to be such that they make it possible to obtain a self-supporting composition, for example in the form of a cleavable stick. In practice, the quantity of polymer (in active substance) represents from 0.5 to 80% of the total weight of the composition, and better from 5 to 40%. The quantity of amphiphilic compound in practice represents from 0.1% to 35% of the total weight of the composition, for example from 1% to 20% and better from 2% to 15%.

According to the invention, it furthermore is preferable that the quantity of film-forming silicone resin be smaller than the quantity of structuring silicone polymer.

Generally, the silicone polymer/film-forming silicone resin mass ratio lies in the range from 20 to 0.15 and better from 15 to 1.5.

The structuring silicone polymer preferably represents 5 to 30% by weight of the composition. The silicone resin preferably represents 0.5 to 20% by weight of the composition.

### Other additives

In addition, the composition of the invention can comprise any ingredient usually used in the field concerned, and in particular those chosen from among the dyes soluble in polyols or in the fatty phase, antioxidants, essential oils, preservatives, fragrances, liposoluble polymers, in particular hydrocarbons such as polyalkylenes or vinyl polylaurate, the gelling agents of the liquid fatty phase, gums, resins, surfactants such as tri-oleyl phosphate, additional cosmetic or dermatological active substances such as water for example, emollients, moisturizers, vitamins, liquid lanolin, essential fatty acids, sunscreens which are lipophilic or soluble in polyols, and mixtures thereof. The composition according to the invention also can contain lipid vesicles of the ionic and/or non-ionic type. These ingredients, except for water, can be present in the composition usually in a proportion from 0 to 20%, preferably from 0.01 to 20%, of the total, weight of the composition, and better from 0.1 to 10%.

In the event that the composition contains an aqueous phase, which is the case for a simple or multiple emulsion, this aqueous phase can represent from 0.1 to 70% of the total weight of the composition, in particular from 0.5 to 40% and better from 1 to 20%. This aqueous phase can contain any compound miscible with water, such as polyols, and possibly be gelled with a suitable gelling agent.

Of course, the person skilled in the art will make sure to choose the possible additional ingredients and/or the quantity thereof in such manner that the advantageous properties of the composition according to the invention are not, or are not substantially, impaired by the contemplated addition.

The composition according to the invention may exist in the form of a dermatological or care composition, possibly tinted, for keratinous matter such as the skin, the lips and/or the phaneric structures, in the form of a composition for sun protection, body hygiene or care, in particular in the form of a deodorant or makeup-removal product in stick or case form. It can be used in particular as a care base for the skin, the phaneric structures or the lips (lip balms, protecting the lips against cold and/or sun and/or wind, a care cream for the skin, nails or hair).

The composition of the invention can exist, in particular, in the form of a rigid gel, especially an anhydrous transparent stick.

The composition of the invention also may exist in the form of a colored makeup product for the skin, in particular a foundation, possibly having care or treatment properties, a blusher, a face powder or eye shadow, a concealer product, an eyeliner, a makeup product for the body; makeup for the lips such as a lipstick, a lip gloss or a pencil possibly having care or treatment properties; makeup for the phaneric structures such as the nails, the eyelashes, in particular in the form of a cake mascara, the eyebrows and the hair, particularly in the form of a pencil.

In particular, the composition of the invention can be a cosmetic product containing cosmetic and/or dermatological active substances such as essential oils, vitamins, moisturizers, filters, healing agents, ceramides.

In the case of makeup compositions, the hydrophobic or hydrophilic solid particles can constitute the pigment(s) making it possible to make up the skin, lips and/or phaneric structures.

Of course, the composition of the invention should be cosmetically or dermatologically acceptable, that is, contain a non-toxic physiologically acceptable medium able to be applied to the skin, phaneric structures or lips of humans. In the meaning of the invention, cosmetically acceptable is understood as a composition with pleasant appearance, odor and feel.

According to the invention, the composition furthermore can contain a coloring agent which can be chosen from among the lipophilic dyes, hydrophilic dyes, and mixtures thereof.

This coloring agent generally is present in a proportion from 0.01 to 50% of the total weight of the composition, preferably from 5 to 30%, if it is present. It is to be noted that a coloring effect also can be provided by the pigments (and nacreous pigments) already described above in the context of the solid particles.

The liposoluble dyes are, for example, Sudan Red, D&C Red 17, D&C Green 6, β-carotene, soybean oil, Sudan Brown, D&C Yellow 11, D&C Violet 2, D&C Orange 5, quinoline yellow or annatto.

The hydrophilic dyes are, in particular, beetroot juice and methylene blue. The soluble dyes can represent from 0 to 20% of the weight of the composition and better from 0.1 to 6% (if present).

The composition according to the invention can be manufactured by known processes generally used in the cosmetic or dermatological field. It can be manufactured by the process which consists in heating the polymer at least to its softening point, adding the oil(s), the film-forming silicone resin or resins and, if necessary, the amphiphilic compound or compounds thereto, then mixing the whole until a clear solution is obtained. There then are added, with stirring, the coloring agents and/or solid particles and the additives. The homogeneous mixture obtained then can be cast in a suitable mold such as a lipstick mold or directly into packaging articles (case or dish in particular).

The invention also has as a purpose a composition characterized in that it is a structured solid composition for making-up of the skin, the lips and/or phaneric structures containing at least one pigment in sufficient quantity for making up the skin, lips and/or phaneric structures, and in that the liquid fatty phase consists in whole or in part of silicone oil(s), said composition existing in the form of a solid, and the pigment, the liquid fatty phase, the film-forming silicone resin and the polymer forming a physiologically acceptable medium.

This makeup composition preferably is self-supporting.

The invention also relates to a composition characterized in that it is a structured composition for lipstick, containing at least one pigment in sufficient quantity for making up the lips, and in that the liquid fatty phase consisting in whole or in part of silicone oil(s),
said composition existing in the form of a solid, and the pigment, the liquid fatty phase and the polymer forming a physiologically acceptable medium.

The composition of the invention also can exist in the form of a cake mascara, an eyeliner, a foundation, a lipstick, a blusher, a makeup-removal product, a makeup product for the body, an eye shadow or face powder, a concealer.

The invention also has as a purpose a composition characterized in that it is a makeup stick for the skin, lips and/or phaneric structures, and in particular the lips, containing at least one pigment in sufficient quantity for making up the skin, lips and/or phaneric structures, and in that the liquid fatty phase consisting in whole or in part of silicone oil(s); the pigment, the fatty phase and the polymer forming a physiologically acceptable medium.

The invention relates to a cosmetic process for care, makeup or treatment of keratinous matter in humans, consisting in the application to the keratinous matter of a cosmetic composition according to the invention.

### Detailed presentation of the invention

The invention is illustrated in greater detail in the following example of formulation of makeup. The quantities are given as % by mass. The chemical compounds are given mainly as the CTFA name ("International Cosmetic Ingredient Dictionary").

### Example 1: Lip gloss

| Code | Composition (nom CTFA) | % |
|---|---|---|
| Phase A | Dimethicone (20 cSt) | qsp 100 |
| | Polyglyceryl-2 diisostearate | 10.0 |
| | Diisostearyl malate | 6.3 |
| | Phenyltrimethicone I (20 cSt) | 10.0 |
| | Phenyltrimethicone II (1000 cSt) | 10.0 |
| | Isononyl isononanoate | 15.5 |
| | Dimethicone and Trimethylsiloxysilicate (MQ resin) | 10.0 |
| Phase B | Polyamidodimethylsiloxane (DP 15) | 16.0 |
| Phase C | Pigments | 1.2 |

This lip gloss is obtained by heating the silicone polyamide to its softening point, then adding the hydrocarbon oils, a portion of the non-volatile silicone oils and the silicone resin. Similarly, the pigments and the other portion of the non-volatile silicone oils are mixed at room temperature, and the whole is subjected to a three-cylinder grinder. The ground material obtained is added to the silicone polyamide mixture obtained, then the whole is homogenized. The mixture then is cast in an appropriate mold.

The product obtained in this manner has, in particular excellent staying-power properties due to the combination, according to the invention, of a film-forming silicone resin and a special silicone polymer.

### REFERENCES

[1] EP-A-1 068 856
[2] WO-A-01/97758
[3] US-A-5 874 069
[4] US-A-5 919 441
[5] US-A-6 051 216
[6] WO-A-02/17870
[7] WO-A-02/17871
[8] EP-A-1 177 784
[9] US-A-5 412 004
[10] EP-A-1 048 686
[11] US-A-5 981 680
[12] WO-A-99/06473
[13] US-A-6 353 076.
[14] EP-A-1 266 653
[15] EP-A-1 266 648
[16] US-A-6 060 072

## Claims

1. Cosmetic composition for makeup, **characterized in that** it comprises:
1)a liquid fatty phase comprising at least one silicone oil, structured with a gelling system comprising at least one polymer (homopolymer or copolymer) with a weight-average molecular mass ranging from 500 to 500,000, containing at least one moiety comprising:
- at least one polyorganosiloxane group, composed of 1 to 1000 organosiloxane units in the chain of the moiety or in the form of a graft, and
- at least two groups capable of establishing hydrogen interactions, said groups being amide groups of formula -C(O)NH- and -HN-C(O)-,
the polymer being solid at room temperature and soluble in the liquid fatty phase at a temperature of 25 to 250°C, and 2) at least one film-forming silicone resin,
the liquid fatty phase, the gelling system and the film-forming silicone resin forming a physiologically acceptable medium,
said polymer comprising at least one moiety of formula(III) 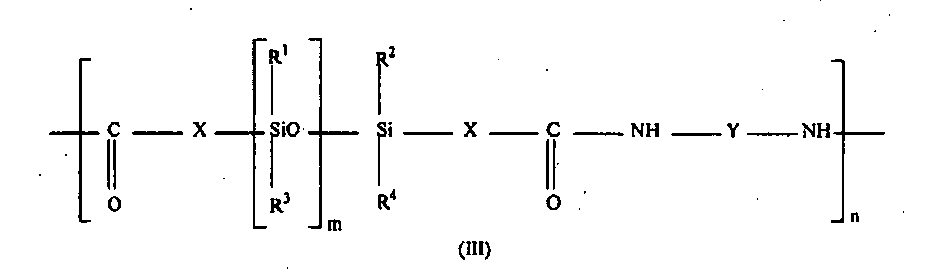
where :
- m is in the range from 1 to 700, preferably from 15 to 500 and n in particular is in the range from 1 to 500, preferably from 1 to 100 and better still from 4 to 25,
- X is a linear or branched alkylene chain having 1 to 30 carbon atoms, in particular 3 to 10 carbon atoms,
- Y is an alkylene chain that is linear or branched or that can comprise rings and/or unsaturations, having from 1 to 40 carbon atoms, in particular from 1 to 20 carbon atoms, and better still from 2 to 6 carbon atoms, in particular 6 carbon atoms, and
- R¹, R², R³ and R⁴ represent, independently, a linear or branched, C1 to C40 alkyl group, preferably a CH3, C2H5, n-C3H7 or isopropyl group, a polyorganosiloxane chain or a phenyl group possibly substituted with one to three methyl or ethyl groups,
said cosmetic composition being further **characterized in that** the film-forming silicone resin is a trimethylsiloxysilicate comprising repeating moieties of formula
[(CH₃)₃-Si-O]ₓ-(SiO_{4/2})y (MQ)
in which x ranges from 50 to 80 and y ranges from 50 to 80,
and said cosmetic composition further comprising a colouring agent.

2. Composition according to claim 1, **characterized in that** the liquid fatty phase comprises at least one volatile silicone oil.

3. Composition according to claim 1 or 2, **characterized in that** the liquid fatty phase comprises at least one volatile silicone oil and at least one volatile non-silicone oil.

4. Composition according to claim 2 or 3, **characterized in that** the volatile silicone oil has a flash point equal to or in excess of 40°C and advantageously in excess of the softening point of the gelling system.

5. Composition according to claim 2, **characterized in that** the fatty phase consists solely of volatile silicone oil(s) having a flash point equal to or in excess of 40°C and advantageously in excess of the softening point of the gelling system.

6. Composition according to any one of claims 2 to 5, **characterized in that** the volatile silicone oil is chosen from within the group consisting of the following compounds: octyltrimethicone, hexyltrimethicone, octamethyl cyclotetrasiloxane D4, dodecamethyl cyclohexasiloxane D6, heptamethyl octyltrisiloxane, decamethyl tetrasiloxane, dodecamethyl pentasiloxane, 1.5 cSt polydimethylsiloxane, 2 cSt polydimethylsiloxane, 3 cSt polymethylsiloxane [sic], 5 cSt polydimethylsiloxane, and mixtures thereof.

7. Composition according to any one of claims 2 to 6, **characterized in that** the volatile oil has a flash point of 40 to 135°C.

8. Composition according to any one of the preceding claims, **characterized in that** the liquid fatty phase also contains a non-volatile silicone oil.

9. Composition according to any one of claims 2 to 8, **characterized in that** the liquid fatty phase contains at least 30% and better still at least 40% by weight of silicone oil.

10. Composition according to any one of claims 2 to 9, **characterized in that** the volatile oil represents from 3 to 89.4%, preferably from 5 to 60%, for example from 5 to 10% of the total weight of the composition.

11. Composition according to any one of claims 1 to 10, **characterized in that** it comprises in addition solid particles chosen from among the fillers; pigments, nacreous or otherwise; and mixtures thereof.

12. Composition according to claim 11, **characterized in that** the solid particles are hydrophobic particles.

13. Composition according to claim 12, **characterized in that** the solid particles are hydrophilic particles, coated in a film of hydrophobic compound.

14. Composition according to claim 11, **characterized in that** the solid particles are hydrophilic particles and the composition further comprises at least one amphiphilic silicone.

15. Composition according to claim 12, **characterized in that** the solid particles consist of powders or fibers of hydrophobic polymers or copolymers.

16. Composition according to one of claims 11 to 15, **characterized in that** the particles are pigments chosen from among the zinc oxides, iron oxides, titanium oxides and mixtures thereof.

17. Composition according to any one of claims 1 to 16, **characterized in that** the polymer used in the gelling system comprises at least one moiety of formula: 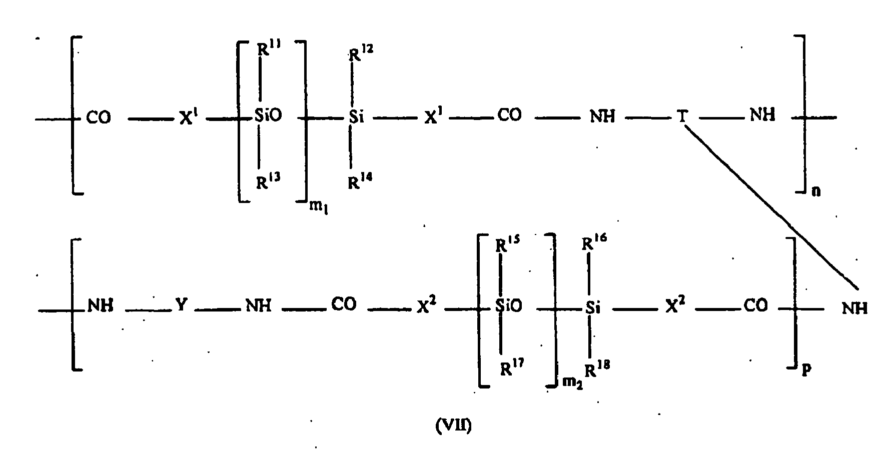 in which X¹ and X² which are identical or different,
represent a linear or branched C₁ to C₃₀ alkylenediyl group, which can contain in its chain one or more oxygen and/or nitrogen atoms,
n is a integer ranging from 2 to 500, preferably from 2 to 200,
Y is a saturated or unsaturated, C₁ to C₅₀ linear or branched divalent alkylene, arylene, cycloalkylene, alkylarylene or arylalkylene group, which can comprise one or more oxygen, sulfur and/or nitrogen atoms, and/or bear as substituent one of the following atoms or groups of atoms: fluorine, hydroxy, C₃ to C₈ cycloalkyl, C₁ to C₄₀ alkyl, C₅ to C₁₀ aryl, phenyl possibly substituted with 1 to 3 C₁ to C₃ alkyl groups, C₁ to C₃ hydroxyalkyl and C₁ to C₆ aminoalkyl, or
4) Y represents a group corresponding to the formula: 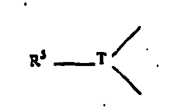 in which
- T represents a linear or branched, saturated or unsaturated, C₃ to C₂₄ trivalent or tetravalent hydrocarbon chain, possibly substituted with a polyorganosiloxane chain, and which can contain one or more atoms chosen from among O, N and S, or T represents a trivalent atom chosen from among N, P and A1, and
- R⁵ represents a linear or branched C₁ to C₅₀ alkyl group, or a polyorganosiloxane chain, which can comprise one or more ester, amide, urethane, thiocarbamate, urea, thiourea and/or sulfonamide groups which can be linked or not linked to another chain of the polymer,
R¹¹ to R¹⁸ are groups chosen from within the same group as R¹ to R⁴, where R¹, R², R³ and R⁴, identical or different, represent a group chosen from among:
- linear, branched or cyclic, saturated or unsaturated, C₁ to C₄₀ hydrocarbon groups, which can contain in their chain one or more oxygen, sulfur and/or nitrogen atoms, and which can be partly or totally substituted with fluorine atoms,
- C₆ to C₁₀ aryl groups, possibly substituted with one or more C₁ to C₄ alkyl groups,
- polyorganosiloxane chains containing or not containing one or more oxygen, sulfur and/or nitrogen atoms;
m₁ and m₂ are numbers falling in the range from 1 to 1000, and p is an integer ranging from 2 to 500.

18. Composition according to the preceding claim, **characterized in that**:
- p is in the range from 1 to 25, better still from 1 to 7,
- R¹¹ to R¹⁸ are methyl groups,
- T corresponds to one of the following formulas: 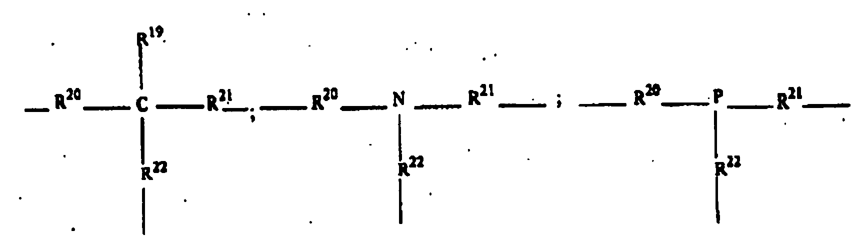 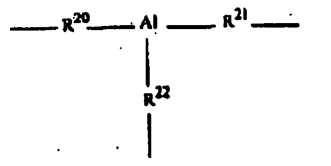 in which R¹⁹ is a hydrogen atom or a group chosen from among the groups defined for R¹ to R₄, and R²⁰, R²¹ and R²² are, independently, linear or branched alkylene groups, and more preferably to the formula: 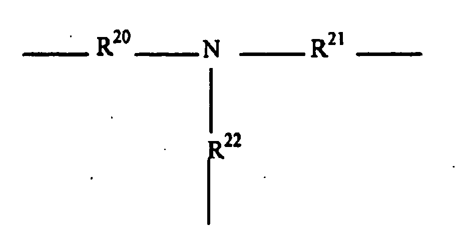
in particular with R²⁰ R²¹ and R²² representing -CH₂-CH₂-
- m₁ and m₂ are in the range from 15 to 500, and better still from 15 to 45,
- X¹ and X² represent -(CH₂)₁₀-, and
- T represents -CH₂-.

19. Composition according to any one of the preceding claims, **characterized in that** the polymer represents from 0.5 to 80% of the total weight of the composition, preferably from 2 to 60% and better from 5 to 40% of the total weight of the composition.

20. Composition according to any one of the preceding claims, **characterized in that** the liquid fatty phase also contains a non-silicone oil.

21. Composition according to any one of the preceding claims, **characterized in that** the liquid fatty phase represents from 5 to 99% of the total weight of the composition, and better from 20 to 75% of the total weight of the composition.

22. Composition according to anyone of the preceding claims, **characterized in that** said at least one film-forming silicone resin is a solid at 25°C.

23. Composition according to anyone of the preceding claims, **characterized in that** said at least one film-forming silicone resin has a weight-average molecular mass ranging from 1000 to 10,000 gram/mole.

24. Composition according to anyone of the preceding claims, **characterized in that** the ratio of the M moieties to the Q moieties is 0.7:1.

25. Composition according to any one of the preceding claims, **characterized in that** said at least one film-forming silicone resin is present in the composition in a quantity ranging from 0.5 to 20%, preferably from 1% to 10% by weight, with respect to the total weight of the composition.

26. Composition according to any one of the preceding claims, **characterized in that** the silicone polymer/film-forming silicone resin mass ratio lies in the range from 20 to 0.15 and better from 15 to 1.5.

27. Composition according to any one of the preceding claims, **characterized in that** it further comprises at least one cosmetic or dermatological active substance.

28. Composition according to the preceding claim, **characterized in that** the active substance is chosen from among the essential oils, vitamins, moisturizers, filters, healing agents and ceramides.

29. Composition according to any one of the preceding claims, **characterized in that** it comprises at least one additive chosen from among the dyes soluble in polyols or in the fatty phase, antioxidants, preservatives, fragrances, liposoluble polymers, in particular hydrocarbons such as the polyalkylenes or vinyl polylaurate, the gelling agents of the liquid fatty phase, gums, resins, surfactants such as tri-oleyl phosphate, additional cosmetic or dermatological active substances chosen, for example, from within the group consisting of water, emollients, moisturizers, vitamins, liquid lanolin, essential fatty acids, sunscreens which are lipophilic or soluble in polyols, lipid vesicles, and mixtures thereof.

30. Composition according to any one of the preceding claims, **characterized in that** it exists in the form of a rigid gel, and in particular an anhydrous stick.

31. Composition according to anyone of the preceding claims, **characterized in that** it is a structured solid composition for making-up of the skin, the lips and/or phaneric structures containing at least one pigment in sufficient quantity for making up the skin, lips and/or phaneric structures,
and **in that**
the liquid fatty phase consists in whole or in part of silicone oil(s),
said composition existing in the form of a solid, and the pigment, the liquid fatty phase, the film-forming silicone resin and the polymer forming a physiologically acceptable medium.

32. Composition according to the preceding claim, **characterized in that** it is self-supporting.

33. Composition according to anyone of the preceding claims, **characterized in that** it is a structured composition for lipstick, containing at least one pigment in sufficient quantity for making up the lips,
and **in that**
the liquid fatty phase consists in whole or in part of silicone oil(s),
said composition existing in the form of a solid, and the pigment, the liquid fatty phase and the polymer forming a physiologically acceptable medium.

34. Composition according to any one of the precedings claims, **characterized in that** it exists in the form of a cake mascara, an eyeliner, a foundation, a lipstick, a blusher, a makeup-removal product, a makeup product for the body, an eye shadow or face powder, a concealer.

35. Composition according to anyone of the preceding claims, **characterized in that** it is a makeup stick for the skin, lips and/or phaneric structures, and in particular the lips, containing at least one pigment in sufficient quantity for making up the skin, lips and/or phaneric structures,
and **in that** the liquid fatty phase consists in whole or in part of silicone oil(s), ; the pigment, the fatty phase, the polymer and the film-forming silicone resin forming a physiologically acceptable medium.

36. Cosmetic process for care, makeup or treatment of keratinous matter in humans, consisting in the application to the keratinous matter of a cosmetic composition according to one of the preceding claims.

## Patentansprüche

1. Kosmetische Zusammensetzung zum Schminken, **dadurch gekennzeichnet, dass** sie folgendes umfasst:
1) eine flüssige Fettphase, die mindestens ein Siliconöl umfasst, das mit einem Geliersystem strukturiert ist, das mindestens ein Polymer (Homopolymer oder Copolymer) mit einer Gewichtsmittel-Molekülmasse im Bereich von 500 bis 500.000, die mindestens eine Einheit enthält, die umfasst:
- mindestens eine Polyorganosiloxangruppe, die aus 1 bis 1000 Organosiloxaneinheiten in der Kette der Einheit oder in der Form einer Pfropfung besteht, und
- mindestens zwei Gruppen, die in der Lage sind, Wasserstoffwechselwirkungen zu entwickeln, wobei die Gruppen Amidgruppen der Formel -C(O)NH- und
- HN-C(O)- sind,
wobei das Polymer bei Raumtemperatur fest und in der flüssigen Fettphase bei einer Temperatur von 25 bis 250 °C löslich ist, und
2) mindestens ein filmbildendes Siliconharz, wobei die flüssige Fettphase, das Geliersystem und das filmbildende Siliconharz ein physiologisch verträgliches Medium bilden,
wobei das Polymer mindestens eine Einheit der Formel (III) umfasst: 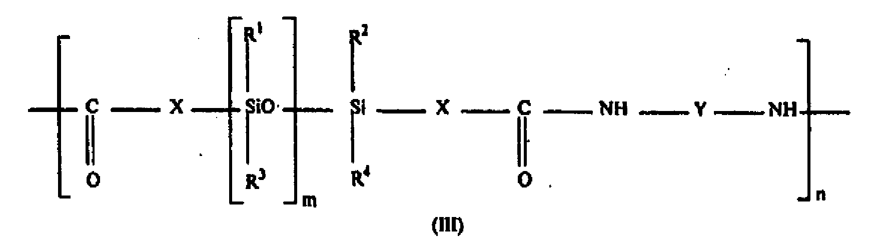 wobei:
- m im Bereich von 1 bis 700 ist, vorzugsweise im Bereich 15 bis 500 ist, und n insbesondere im Bereich von 1 bis 500 ist, vorzugsweise von 1 bis 100 ist und besser noch von 4 bis 25 ist,
- X eine lineare oder verzweigte Alkylenkette mit 1 bis 30 Kohlenstoffatomen, insbesondere mit 3 bis 10 Kohlenstoffatomen ist,
- Y eine Alkylenkette ist, die linear oder verzweigt ist oder die Ringe und/oder Ungesättigtheiten mit 1 bis 40 Kohlenstoffatomen, insbesondere mit 1 bis 20 Kohlenstoffatomen und besser noch mit 2 bis 6 Kohlenstoffatomen, insbesondere mit 6 Kohlenstoffatomen umfassen kann, und
- R¹, R², R³, und R⁴ unabhängig eine lineare oder verzweigte C₁- bis C₄₀-Alkylgruppe darstellen, vorzugsweise eine CH₃-, C₂H₅-, n-C₃H₇- oder Isopropylgruppe, eine Polyorganosiloxankette oder eine Phenylgruppe, die gegebenenfalls mit einer bis drei Methyl- oder Ethylgruppen substituiert ist,
wobei die kosmetische Zusammensetzung weiterhin **dadurch gekennzeichnet ist, dass** das filmbildende Siliconharz ein Trimethylsiloxysilicat ist, das Wiederholungseinheiten der Formel
[(CH₃)₃-Si-O]ₓ-(SiO_{4/2})_{y} (MQ)
umfasst, wobei x von 50 bis 80 reicht und y von 50 bis 80 reicht;
und wobei die kosmetische Zusammensetzung weiterhin ein Färbemittel umfasst.

2. Zusammensetzung nach Anspruch 1, **dadurch gekennzeichnet, dass** die flüssige Fettphase mindestens ein flüchtiges Siliconöl umfasst.

3. Zusammensetzung nach Anspruch 1 oder 2, **dadurch gekennzeichnet, dass** die flüssige Fettphase mindestens ein flüchtiges Siliconöl und mindestens ein flüchtiges Nichtsiliconöl umfasst.

4. Zusammensetzung nach Anspruch 2 oder 3, **dadurch gekennzeichnet, dass** das flüchtige Siliconöl einen Flashpunkt von gleich oder oberhalb von 40 °C und zweckmäßigerweise oberhalb des Erweichungspunkts des Geliersystems umfasst.

5. Zusammensetzung nach Anspruch 2, **dadurch gekennzeichnet, dass** die Fettphase nur aus flüchtigem(n) Siliconöl(en) mit einem Flashpunkt von gleich oder oberhalb von 40 °C und zweckmäßigerweise von oberhalb des Erweichungspunktes des Geliersystems besteht.

6. Zusammensetzung nach einem der Ansprüche 2 bis 5, **dadurch gekennzeichnet, dass** das flüchtige Siliconöl aus der Gruppe ausgewählt ist, die aus den folgenden Verbindungen besteht: Octyltrimethicon, Hexyltrimethicon, Octamethylcyclotetrasiloxan D4, Dodecamethylcyclohexasiloxan D6, Heptamethyloctyltrisiloxan, Decamethyltetrasiloxan, Dodecamethylpentasiloxan, 1,5 cSt Polydimethylsiloxan, 2 cSt Polydimethylsiloxan, 3 cSt Polymethylsiloxan [sic], 5 cSt Polydimethylsiloxan und Gemischen davon.

7. Zusammensetzung nach einem der Ansprüche 2 bis 6, **dadurch gekennzeichnet, dass** das flüchtige Öl einen Flashpunkt von 40 bis 135 °C aufweist.

8. Zusammensetzung nach einem der vorhergehenden Ansprüche, **dadurch gekennzeichnet, dass** die flüssige Fettphase auch ein nicht flüchtiges Siliconöl enthält.

9. Zusammensetzung nach einem der Ansprüche 2 bis 8, **dadurch gekennzeichnet, dass** die flüssige Fettphase mindestens 30 Gew.-% und besser noch mindestens 40 Gew.-% Siliconöl enthält.

10. Zusammensetzung nach einem der Ansprüche 2 bis 9, **dadurch gekennzeichnet, dass** das flüchtige Öl 3 bis 89,4 %, vorzugsweise 5 bis 60 %, beispielsweise 5 bis 10 % des Gesamtgewichts der Zusammensetzung darstellt.

11. Zusammensetzung nach einem der Ansprüche 1 bis 10, **dadurch gekennzeichnet, dass** sie zusätzlich feste Teilchen umfasst, ausgewählt aus Füllstoffen; Pigmenten, perlglanzartig oder anders; und Gemischen davon.

12. Zusammensetzung nach Anspruch 11, **dadurch gekennzeichnet, dass** die festen Teilchen hydrophobe Teilchen sind.

13. Zusammensetzung nach Anspruch 12, **dadurch gekennzeichnet, dass** die festen Teilchen mit einem Film aus einer hydrophoben Verbindung überzogene hydrophile Teilchen sind.

14. Zusammensetzung nach Anspruch 11, **dadurch gekennzeichnet, dass** die festen Teilchen hydrophile Teilchen sind und die Zusammensetzung weiterhin mindestens ein amphiphiles Silicon umfasst.

15. Zusammensetzung nach Anspruch 12, **dadurch gekennzeichnet, dass** die festen Teilchen aus Pulvern oder Fasern von hydrophoben Polymeren oder Copolymeren bestehen.

16. Zusammensetzung nach einem der Ansprüche 11 bis 15, **dadurch gekennzeichnet, dass** die Teilchen Pigmente sind, die aus Zinkoxiden, Eisenoxiden, Titanoxiden und Gemischen davon ausgewählt sind.

17. Zusammensetzung nach einem der Ansprüche 1 bis 16, **dadurch gekennzeichnet, dass** das in dem Geliersystem verwendete Polymer mindestens eine Einheit der folgenden Formel umfasst: 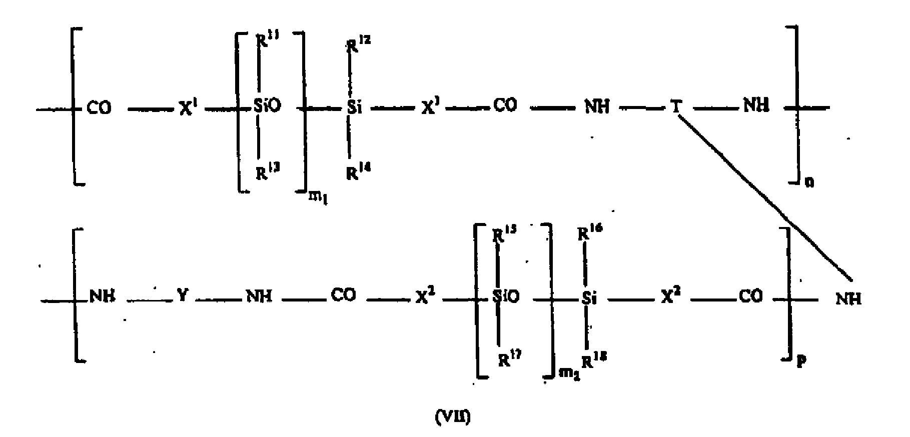 wobei
X¹ und X², die identisch oder verschieden sind, eine lineare oder verzweigte C₁- bis C₃₀-Alkylendiylgruppe darstellen, die in ihrer Kette ein oder mehrere Sauerstoff- und/oder Stickstoffatome enthalten kann, n eine ganze Zahl von 2 bis 500, vorzugsweise von 2 bis 200 ist,
Y eine gesättigte oder ungesättigte C₁- bis C₃₀-lineare oder verzweigte zweiwertige Alkylen-, Arylen-, Cycloalkylen, Alkylarylen- oder Arylalkylengruppe ist, die ein oder mehrere Sauerstoff-, Schwefel- und/oder Stickstoffatome umfassen kann und/oder als Substituent ein oder mehrere der folgenden Atome oder Gruppen von Atomen tragen kann: Fluor, Hydroxy, C₃- bis C₆-Cycloalkyl, C₁- bis C₄₀-Alkyl, C₈- bis C₁₀-Aryl, Phenyl, das gegebenenfalls substituiert ist mit 1 bis 3 C₁- bis C₃-Alkylgruppen, C₁- bis C₃-Hydroxyalkyl und C₁- bis C₆-Aminoalkyl, oder
Y eine Gruppe darstellt, die der folgenden Formel entspricht: 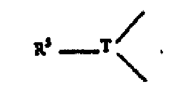 wobei:
- T eine lineare oder verzweigte, gesättigte oder ungesättigte C₃- bis C₂₄-dreiwertige oder -vierwertige Kohlenwasserstoffkette darstellt, die gegebenenfalls mit einer Polyorganosiloxankette substituiert ist, und die ein oder mehrere Atome enthalten kann, die aus O, N und S ausgewählt sind, oder T ein dreiwertiges Atom darstellt, das aus N, P und A1 ausgewählt ist, und
- R⁵ eine lineare oder verzweigte C₁- bis C₃₀-Alkylgruppe oder eine Polyorganosiloxankette darstellt, die eine oder mehrere Ester-, Amid-, Urethan-, Thiocarbamat, Harnstoff, Thioharnstoff und/oder Sulfonamidgruppen umfassen kann, die mit einer weiteren Kette des Polymers verknüpft sein können oder nicht, R¹¹ bis R¹⁸ Gruppen sind, die aus der gleichen Gruppe wie R¹ bis R⁴ ausgewählt sind, wobei R¹, R², R³ und R⁴, identisch oder verschieden, eine aus folgendem ausgewählte Gruppe darstellt:
- lineare, verzweigte oder cyclische, gesättigte oder ungesättigte C₁- bis C₄₀-Kohlenwasserstoffgruppen, die in ihrer Kette ein oder mehrere Sauerstoff-, Schwefel- und/oder Stickstoffatome enthalten können, und die teilweise oder vollständig mit Fluoratomen substituiert sein können,
- C₆- bis C₁₀-Arylgruppen, die gegebenenfalls mit einer oder mehreren C₁- bis C₄-Alkylgruppen substituiert sein können,
- Polyorganosiloxanketten, die ein oder mehrere Sauerstoff-, Schwefel und/oder Stickstoffatome enthalten oder nicht,
m₁ und m₂ Zahlen sind, die in den Bereich von 1 bis 1000 fallen, und p eine ganze Zahl im Bereich von 2 bis 500 ist.

18. Zusammensetzung nach dem vorhergehenden Anspruch, **dadurch gekennzeichnet dass**:
- p im Bereich von 1 bis 25, besser noch im Bereich von 1 bis 7 liegt,
- R¹¹ bis R¹⁸ Methylgruppen sind,
- T einer der folgenden Formeln entspricht: 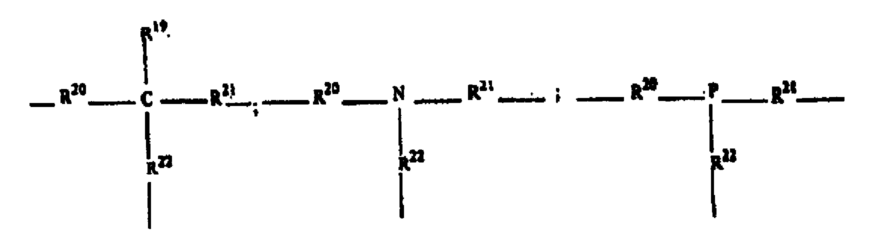 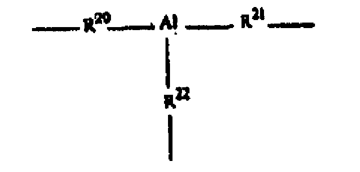 wobei R¹⁹ ein Wasserstoffatom oder eine Gruppe ist, die aus den für R¹ bis R⁴ definierten Gruppen ausgewählt sind, und R²⁰, R²¹ und R²² unabhängig lineare oder verzweigte Alkylengruppen sind, und stärker bevorzugt der folgenden Formel entspricht: 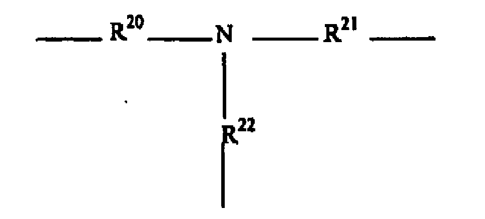 insbesondere wobei R²⁰, R²¹ und R²² -CH₂-CH₂- darstellen,
- m₁ und m₂ im Bereich von 15 bis 500 und besser noch im Bereich von 15 bis 45 sind,
- X¹ und X² -(CH₂)₁₀- darstellen, und
- T -CH₂- darstellt.

19. Zusammensetzung nach einem der vorhergehenden Ansprüche, **dadurch gekennzeichnet, dass** das Polymer 0,5 bis 80 % des Gesamtgewichts der Zusammensetzung, vorzugsweise von 2 bis 60 % und besser von 5 bis 40 % des Gesamtgewicht der Zusammensetzung darstellt.

20. Zusammensetzung nach einem der vorhergehenden Ansprüche, **dadurch gekennzeichnet, dass** die flüssige Fettphase auch ein Nichtsiliconöl enthält.

21. Zusammensetzung nach einem der vorhergehenden Ansprüche, **dadurch gekennzeichnet, dass** die flüssige Fettphase 5 bis 99 % des Gesamtgewichts der Zusammensetzung, und besser von 20 bis 75 % des Gesamtgewicht der Zusammensetzung darstellt.

22. Zusammensetzung nach einem der vorhergehenden Ansprüche, **dadurch gekennzeichnet, dass** das mindestens eine filmbildende Siliconharz bei 25 °C fest ist.

23. Zusammensetzung nach einem der vorhergehenden Ansprüche, **dadurch gekennzeichnet, dass** das mindestens eine filmbildende Siliconharz eine Gewichtsmittel-Molekülmasse im Bereich von 1000 bis 10.000 g/mol besitzt.

24. Zusammensetzung nach einem der vorhergehenden Ansprüche, **dadurch gekennzeichnet, dass** das Verhältnis der M-Einheiten zu den Q-Einheiten 0,7:1 beträgt.

25. Zusammensetzung nach einem der vorhergehenden Ansprüche, **dadurch gekennzeichnet, dass** das mindestens eine filmbildende Siliconharz in der Zusammensetzung in einer Menge im Bereich von 0,5 bis 20 Gew.-%, vorzugsweise von 1 bis 10 Gew.-% bezüglich des Gesamtgewicht der Zusammensetzung vorhanden ist.

26. Zusammensetzung nach einem der vorhergehenden Ansprüche, **dadurch gekennzeichnet, dass** das Siliconpolymer/filmbildende Siliconharz-Masseverhältnis im Bereich von 20 bis 0,15 und besser von 15 bis 1,5 liegt.

27. Zusammensetzung nach einem der vorhergehenden Ansprüche, **dadurch gekennzeichnet, dass** sie weiterhin mindestens eine kosmetisch oder dermatologisch wirksame Substanz umfasst.

28. Zusammensetzung nach einem der vorhergehenden Ansprüche, **dadurch gekennzeichnet, dass** die wirksame Substanz aus ätherischen Ölen, Vitaminen, Befeuchtem, Filtern, Heilmitteln und Ceramiden ausgewählt ist.

29. Zusammensetzung nach einem der vorhergehenden Ansprüche, **dadurch gekennzeichnet, dass** sie mindestens ein Additiv umfasst, ausgewählt aus den in Polyolen oder in der Fettphase löslichen Farbstoffen, Antioxidantien, Konservierungsmitteln, Duftstoffen, fettlöslichen Polymeren, insbesondere Kohlenwasserstoffen, wie Polyalkylene oder Vinylpolylaureat, den Geliermitteln der flüssigen Fettphase, Gummen, Harzen, grenzflächenaktiven Mitteln, wie Trioleylphosphat, zusätzlichen kosmetisch oder dermatologisch wirksamen Substanzen, ausgewählt beispielsweise aus der Gruppe, bestehend aus Wasser, Emollientien, Befeuchtern, Vitaminen, flüssigem Lanolin, essentiellen Fettsäuren, Sonnenschutzmitteln, die lipophil oder in Polyolen löslich sind, Lipidvesikeln und Gemischen davon.

30. Zusammensetzung nach einem der vorhergehenden Ansprüche, **dadurch gekennzeichnet, dass** sie in der Form eines starren Gels und insbesondere als ein wasserfreier Stift existiert.

31. Zusammensetzung nach einem der vorhergehenden Ansprüche, **dadurch gekennzeichnet, dass** sie in einer strukturierten festen Zusammensetzung zum Schminken der Haut, der Lippen und/oder der phaneren Strukturen vorliegt, die mindestens ein Pigment in ausreichender Menge zum Schminken der Haut, der Lippen und/oder der phaneren Strukturen enthält,
und **dadurch**, dass
die flüssige Fettphase insgesamt oder teilweise aus Siliconöl(en) besteht,
wobei die Zusammensetzung in der Form eines Feststoffs existiert, und das Pigment, die flüssige Fettphase, das filmbildende Siliconharz und das Polymer ein physiologisch verträgliches Medium bilden.

32. Zusammensetzung nach dem vorhergehenden Anspruch, **dadurch gekennzeichnet, dass** sie selbsttragend ist.

33. Zusammensetzung nach einem der vorhergehenden Ansprüche, **dadurch gekennzeichnet, dass** sie eine strukturierte feste Zusammensetzung für Lippenstift ist, die mindestens ein Pigment in ausreichender Menge zum Schminken der Lippen enthält,
und **dadurch**, dass
die flüssige Fettphase insgesamt oder teilweise aus Siliconöl(en) besteht,
wobei die Zusammensetzung in der Form eines Feststoffs existiert, und das Pigment, die flüssige Fettphase, und das Polymer ein physiologisch verträgliches Medium bilden.

34. Zusammensetzung nach einem der vorhergehenden Ansprüche, **dadurch gekennzeichnet, dass** sie in der Form eines Mascarablocks, eines Eyeliners, einer Grundierung, eines Lippenstifts, eines Wangenrouge, eines Make-up-Entfemungsprodukts, eines Schminkprodukts für den Körper, eines Lidschattens oder Gesichtspuders, eines Concealers vorliegt.

35. Zusammensetzung nach einem der vorhergehenden Ansprüche, **dadurch gekennzeichnet, dass** sie ein Schminkstift für die Haut, die Lippen und/oder die phaneren Strukturen ist, und insbesondere der Lippen ist, der mindestens ein Pigment in ausreichender Menge zum Schminken der Haut, der Lippen und/oder der phaneren Strukturen enthält,
und **dadurch**, dass
die flüssige Fettphase insgesamt oder teilweise aus Siliconöl(en) besteht, wobei das Pigment, die Fettphase, das Polymer und das filmbildende Siliconharz ein physiologisch verträgliches Medium bilden.

36. Kosmetisches Verfahren zur Pflege, Schminke oder zur Behandlung von keratinischer Substanz in Menschen, bestehend aus dem Auftragen auf die keratinische Substanz einer kosmetischen Zusammensetzung nach einem der vorhergehenden Ansprüche.

## Revendications

1. Composition cosmétique de maquillage, **caractérisée en ce qu'**elle comprend :
1) une phase grasse liquide comprenant au moins une huile de silicone, structurée avec un système gélifiant comprenant au moins un polymère (homopolymère ou copolymère) ayant une masse moléculaire moyenne en poids allant dé 500 à 500 000, qui contient au moins une fraction comprenant :
- au moins un groupe polyorganosiloxane, composé de 1 à 1 000 unités organosiloxane dans'la chaîne de la fraction ou sous la forme d'une greffe, et
- au moins deux groupes capables d'établir des interactions hydrogène, lesdits groupes étant des groupes amide de formule -C(O)NH- et -HN-C(O)-,
le polymère étant solide à température ambiante et soluble dans la phase grasse liquide à une température de 25 à 250°C, et
2) au moins une résine de silicone filmogène, la phase grasse liquide, le système gélifiant et la résine de silicone filmogène formant un milieu physiologiquement acceptable,
ledit polymère comprenant au moins une fraction de formule (III) : 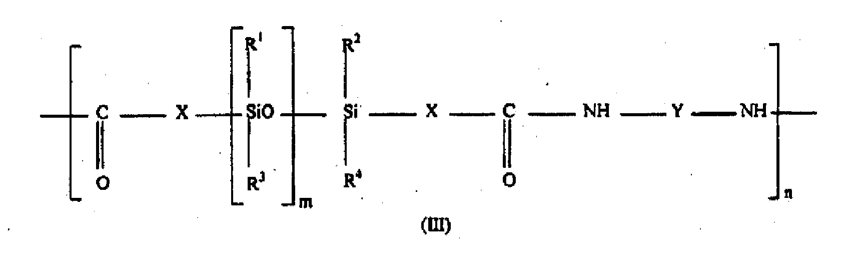 dans laquelle :
- m va de 1 à 700, de préférence de 15 à 500 et n en particulier va de 1 à 500, de préférence de 1 à 100 et mieux encore de 4 à 25,
- x est une chaîne alkylène linéaire ou ramifiée ayant 1 à 30 atomes de carbone, en particulier 3 à 10 atomes de carbone,
- y est une chaîne alkylène qui est linéaire ou ramifiée ou qui peut comprendre des cycles et/ou des in-saturations, ayant de 1 à 40 atomes de carbone, en particulier de 1 à 20 atomes de carbone, et mieux encore de 2 à 6 atomes de carbone, en particulier 6 atomes de carbone, et
- R¹, R², R³ et R⁴ représentent, indépendamment, un groupe alkyle en C₁ - C₄₀, linéaire ou ramifié, de préférence un groupe CH3, C2H5, n-C3H7 ou isopropyle, une chaîne polyorganosiloxane ou un groupe phényle éventuellement substitué avec un à trois groupes méthyle ou éthyle,
ladite composition cosmétique étant en outre **caractérisée en ce que** la résine de silicone filmogène est un triméthylsiloxysilicate comprenant des fractions répétées de formule
[(CH₃)₃-Si-O]ₓ-(SiO_{4/2})y (MQ);
dans laquelle x va de 50 à 80 et y va de 50 à 80,
et ladite composition cosmétique comprend en outre un agent colorant.

2. Composition selon la revendication 1, **caractérisée en ce que** la phase grasse liquide comprend au moins une huile de silicone volatile.

3. Composition selon la revendication 1 ou 2, **caractérisée en ce que** la phase grasse liquide comprend au moins une huile de silicone volatile et au moins une huile de silicone non volatile.

4. Composition selon la revendication 2 ou 3, **caractérisée en ce que** l'huile de silicone volatile a un point d'éclair égal ou supérieur à 40°C et avantageusement supérieur au point de ramollissement du système gélifiant.

5. Composition selon la revendication 2, **caractérisée en ce que** la phase grasse est constituée uniquement d'huile(s) de silicone volatile(s) ayant un point d'éclair égal ou supérieur à 40°C et avantageusement supérieur au point de ramollissement du système gélifiant.

6. Composition selon l'une quelconque des revendications 2 à 5, **caractérisée en ce que** l'huile de silicone volatile est choisie parmi le groupe constitué des composés suivants : octyltriméthicone, hexyltriméthicone, octaméthyl-cyclotétrasiloxane D4, dodécaméthylcyclohexasiloxane D6, heptaméthyl-octyltrisiloxane, décaméthyl-tétrasiloxane, dodécaméthyl-pentasiloxane, 1.5 cSt polydiméthylsiloxane, 2 cSt polydiméthylsiloxane, 3 cSt polyméthylsiloxane [sic], 5 cSt polydiméthylsiloxane, et des mélanges de ceux-ci.

7. Composition selon l'une quelconque des revendications 2 à 6, **caractérisée en ce que** l'huile volatile a un point d'éclair de 40 à 135°C.

8. Composition selon l'une quelconque des revendications précédentes, **caractérisée en ce que** la phase grasse liquide contient également une huile de silicone non volatile.

9. Composition selon l'une quelconque des revendications 2 à 8, **caractérisée en ce que** la phase grasse liquide contient au moins 30 % et mieux encore au moins 40 % en poids d'huile de silicone.

10. Composition selon l'une quelconque des revendications 2 à 9, **caractérisée en ce que** l'huile volatile représente de 3 à 89,4 %, de préférence de 5 à 60 %, par exemple de 5 à 10 % du poids total de la composition.

11. Composition selon l'une quelconque des revendications 1 à 10, **caractérisée en ce qu'**elle comprend en outre des particules solides choisies parmi les charges ; pigments, nacres ou autres ; et des mélanges de ceux-ci.

12. Composition selon la revendication 11, **caractérisée en ce que** les particules solides sont des particules hydrophobes.

13. Composition selon la revendication 12, **caractérisée en ce que** les particules solides sont des particules hydrophiles, enrobées dans un film d'un composé hydrophobe.

14. Composition selon la revendication 11, **caractérisée en ce que** les particules solides sont des particules hydrophiles et la composition comprend en outre au moins un silicone amphiphile.

15. Composition selon la revendications 12, **caractérisée en ce que** les particules solides sont constituées de poudres ou'de fibres de polymères ou copolymères hydrophobes.

16. Composition selon l'une des revendications 11 à 15, **caractérisée en ce que** les particules sont des pigments choisis parmi les oxydes de zinc, oxydes de fer, oxydes de titane et des mélanges de ceux-ci.

17. Composition selon l'une quelconque des revendications 1 à 16, **caractérisée en ce que** le polymère utilisé dans le système gélifiant comprend au moins une fraction de formule : 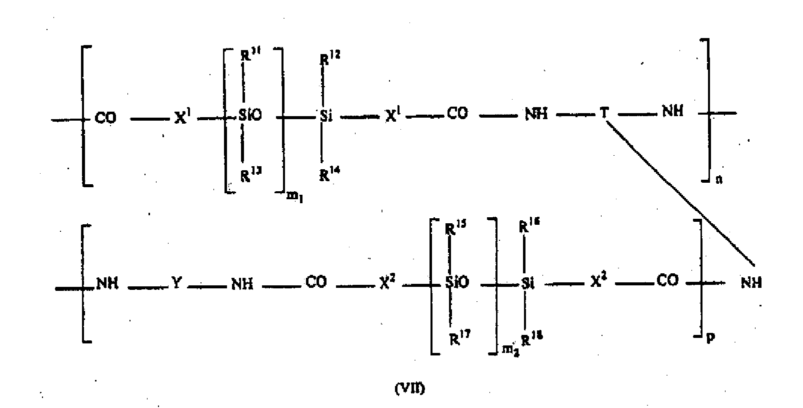 dans laquelle X¹ et X², qui sont identiques ou différents, représentent un groupe alkylènediyle en C₁-C₃₀ linéaire ou ramifié, qui peut contenir dans sa chaîne un ou plusieurs atomes d'oxygène et/ou d'azote, n est un nombre entier allant de 2 à 500, de préférence de 2 à 200, Y est un groupe alkylène, arylène, cycloalkylène, alkylarylène ou arylalkylène divalent linéaire ou ramifié en C₁-C₅₀, saturé ou insaturé, qui peut comprendre un ou plusieurs atomes d'oxygène, de soufre et/ou d'azote, et/ou porter comme substituant l'un des atomes ou groupes d'atomes suivants : fluor, hydroxy, cycloalkyle en C₃-C₈, alkyle en C₁-C₄₀, aryle en C₅-C₁₀, phényle éventuellement substitué avec 1 à 3 groupes alkyle en C₁-C₃, hydroxyalkyle en C₁-C₃ et aminoalkyle en C₁-C₆, ou
Y représente un groupe correspondant à la formule : 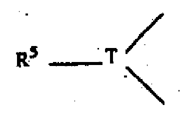 dans laquelle
- T représente une chaîne hydrocarbonée linéaire ou ramifiée, saturée ou insaturée, trivalente ou tétravalente en C₃-C₂₄, éventuellement substituée avec une chaîne polyorganosiloxane, et qui peut contenir un ou plusieurs atomes choisis parmi o, N et S, ou T représente un atome trivalent choisi parmi N, P et Al, et
- R⁵ représente un groupe alkyle en C₁-C₅₀ linéaire ou ramifié, ou une chaîne polyorganosiloxane, qui peut comprendre un ou plusieurs groupes ester, amide, uréthane, thiocarbamate, urée, thiourée et/ou sulfonamide qui peuvent être liés ou ne peuvent pas être liés à une autre chaîne du polymère,
R¹¹ à R¹⁸ sont des groupes choisis parmi le même groupe que R¹ à R⁴ dans lequel R¹, R², R³ et R⁴ identiques ou différents, représentent un groupe choisi parmi :
- des groupes hydrocarbonés en C₁-C₄₀ linéaires, ramifiés ou cycliques, saturés ou insaturés, qui peuvent contenir dans leur chaîne un ou plusieurs atomes d'oxygène, de soufre et/ou d'azote, et qui peuvent être substitués en partie ou en totalité avec des atomes de fluor,
- des groupes aryle en C₆-C₁₀ éventuellement substitués avec un ou plusieurs groupes alkyle en C₁-C₄,
- des chaînes de polyorganosiloxane contenant ou ne contenant pas un ou plusieurs atomes d'oxygène, de soufre et/ou d'azote ;
m₁ et m₂ sont des nombres allant de 1 à 1 000, et p est un nombre entier allant de 2 à 500.

18. Composition selon la revendication précédente, **caractérisée en ce que :**
- p va de 1 à 25 et mieux encore de 1 à 7,
- R¹¹ à R¹⁸ sont des groupes méthyle,
- T correspond à l'une des formules suivantes : 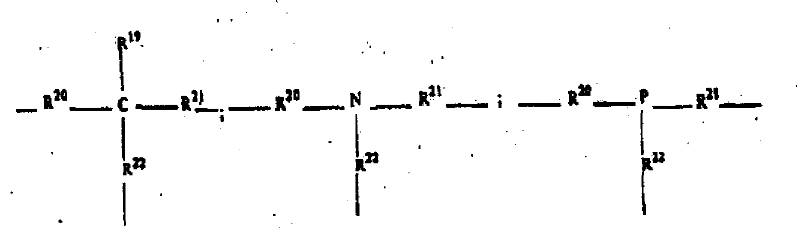 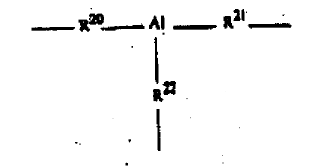 dans lesquelles R¹⁹ est un atome d'hydrogène ou un groupe choisi parmi les groupes définis pour R¹ à R⁴, et R²⁰, R²¹ et R²² sont, indépendamment, des groupes alkylène linéaires ou ramifiés, et plus avantageusement correspond à la formule : 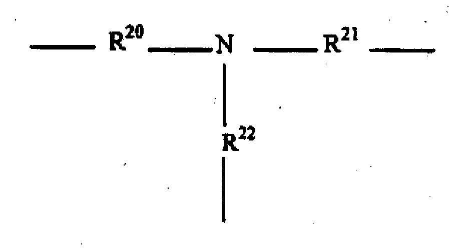 en particulier, avec R²⁰, R²¹ et R²² représentant -CH₂-CH₂-,
- m₁ et m₂ vont de 15 à 500 et mieux encore de 15 à 45,
- X¹ et X² représentent -(CH₂)₁₀-, et
- T représente -CH₂-.

19. Composition selon l'une quelconque des revendications précédentes, **caractérisée en ce que** le polymère représente de 0,5 à 80 % du poids total de la composition, de préférence de 2 à 60 % et mieux encore de 5 à 40 % du poids total de la composition.

20. Composition selon l'une quelconque des revendications précédentes, **caractérisée en ce que** la phase grasse liquide contient également une huile non siliconée.

21. Composition selon l'une quelconque des revendications précédentes, **caractérisée en ce que** la phase grasse liquide représente de 5 à 99 % du poids total de la composition, et de préférence de 20 à 75 % du poids total de la composition.

22. Composition selon l'une quelconque des revendications précédente, **caractérisée en ce que** ladite au moins une résine de silicone filmogène est un solide à 25°C.

23. Composition selon l'une quelconque des revendications précédentes, **caractérisée en ce que** ladite au moins une résine de silicone filmogène a une masse moléculaire moyenne en poids allant de 1 000 à 10 000 grammes/mole.

24. Composition selon l'une quelconque des revendications précédentes, **caractérisée en ce que** le rapport des fractions M sur les fractions Q est 0,7:1.

25. Composition selon l'une quelconque des revendications précédentes, **caractérisée en ce que** ladite au moins une résine de silicone filmogène est présente dans la composition en une quantité allant de 0,5 à 20 %, de préférence de 1 % à 10 % en poids, par rapport au poids total de la composition.

26. Composition selon l'une quelconque des revendications précédentes, **caractérisée en ce que** le rapport massique polymère de silicone/résine de silicone filmogène va de 20 à 0,15 et de préférence de 15 à 1,5.

27. Composition selon l'une quelconque des revendications précédentes, **caractérisée en ce qu'**elle contient en outre au moins un principe actif cosmétique ou dermatologique.

28. Composition selon la revendication précédente, **caractérisée en ce que** le principe actif est choisi parmi les huiles essentielles, des vitamines, des humectants, des filtres, des agents cicatrisants et des céramides.

29. Composition selon l'une quelconque des revendications précédentes, **caractérisée en ce qu'**elle comprend au moins un additif choisi parmi les colorants solubles dans des polyols ou dans la phase grasse, des antioxydants, des agents de conservation, des parfums, des polymères liposolubles, en particulier à des hydrates de carbone, tels que les polyalkylènes ou le polylaurate de vinyle, des agents gélifiants de la phase grasse liquide, des gommes, des résines, des tensioactifs tels que le phosphate de trioléyle, des principes actifs cosmétiques ou dermatologiques supplémentaires choisis, par exemple, parmi le groupe constitué de l'eau, d'émollients, d'humectants, de vitamines, de lanoline liquide, d'acides gras essentiels, de crèmes solaires qui sont lipophiles ou solubles dans des polyols, des vésicules lipidiques, et des mélanges de ceux-ci.

30. Composition selon l'une quelconque des revendications précédentes, **caractérisée en ce qu'**elle existe sous la forme d'un gel rigide, et en particulier d'un stick anhydre.

31. Composition selon l'une quelconque des revendications précédentes, **caractérisée en ce qu'**elle est une composition solide structurée pour maquillage de la peau, des lèvre et/ou des structures phanériennes contenant au moins un pigment en quantité suffisante pour le maquillage de la peau, des lèvres et/ou des structures phanériennes,
et **en ce que**
la phase grasse liquide est constituée en tout ou en partie d'huile(s) de silicone,
ladite composition existant sous la forme d'un solide, et le pigment, la phase grasse liquide, la résine de silicone filmogène et le polymère formant un milieu physiologiquement acceptable.

32. Composition selon la revendication précédente, **caractérisée en ce qu'**elle est autoporteuse.

33. Composition selon l'une quelconque des revendications précédentes, **caractérisée en ce qu'**elle est une composition structurée pour stick de rouge à lèvres, contenant au moins un pigment en quantité suffisante pour maquiller les lèvres,
et **en ce que**
la phase grasse liquide est constituée en tout ou en partie d'huile(s) de silicone,
ladite composition existant sous la forme d'un solide, et le pigment, la phase grasse liquide et le polymère formant un milieu physiologiquement acceptable.

34. Composition selon l'une quelconque des revendications précédentes, **caractérisée en ce qu'**elle-existe sous la forme d'un mascara pain, d'un eye-liner, d'un fond de teint, d'un stick de rouge à lèvres, d'un blush, d'un démaquillant, d'un produit de maquillage du corps; d'un fard à paupières ou d'un fard à joues, ou d'un produit anticerne.

35. Composition selon l'une quelconque des revendications précédentes, **caractérisée en ce qu'**elle est un stick de maquillage pour la peau, les lèvres et/ou les structures phanériennes, et en particulier les lèvres, contenant au moins un pigment en quantité suffisante pour maquiller la peau, les lèvres et/ou les structures phanériennes,
et **en ce que** la phase grasse liquide est constituée en tout ou en partie d'huile(s) de silicone ; le pigment, la phase grasse, le polymère et la résine de silicone filmogène formant un milieu physiologiquement acceptable.

36. Procédé cosmétique pour soin, maquillage ou traitement d'une matière kératineuse chez l'homme, consistant en l'application sur la matière kératineuse d'une composition cosmétique selon l'une des revendications précédentes.
